# EUROPEAN PATENT SPECIFICATION

(11) **EP 3 256 453 B1**
(45) Date of publication and mention of the grant of the patent: **31.08.2022**
(21) Application number: 16749359.2
(22) Date of filing: 21.01.2016
(51) Int. Cl.: C07D 251/24, C07D 307/91, C07D 333/76, C07C 13/567, C09K 11/06, H01L 51/54

(54) **ORGANIC ELECTROLUMINESCENT DEVICE**
ORGANISCHE ELEKTROLUMINESZENZVORRICHTUNG
DISPOSITIF ÉLECTROLUMINESCENT ORGANIQUE

(30) Priority: 12.02.2015 KR 20150021675; 07.01.2016 KR 20160002161
(43) Date of publication of application: 20.12.2017
(73) Proprietor: Rohm And Haas Electronic Materials Korea Ltd., Cheonan-si, Chungcheongnam-do 31093 (KR)
(72) Inventor: KANG, Hee-Ryong, Hwaseong-si, Gyeonggi-Do 18449 (KR); HONG, Jin Ri, Hwaseong-si, Gyeonggi-Do 18449 (KR); KIM, Bitnari, Hwaseong-si, Gyeonggi-Do 18449 (KR); CHO, Sang Hee, Hwaseong-si, Gyeonggi-Do 18449 (KR)
(74) Representative: Houghton, Mark Phillip
(86) International application number: PCT/KR2016/000666
(87) International publication number: WO 2016/129819

(56) References cited:
- EP-A1- 3 129 446
- WO-A1-2013/105747
- WO-A1-2013/105747
- WO-A1-2014/104720
- WO-A1-2015/156587
- WO-A1-2015/174738
- WO-A2-2013/122364

## Description

### Technical Field

The present invention relates to an organic electroluminescent device.

### Background Art

An electroluminescent device (EL device) is a self-light-emitting device which has advantages in that it provides a wider viewing angle, a greater contrast ratio, and a faster response time. The first organic EL device was developed by Eastman Kodak, by using small aromatic diamine molecules, and aluminum complexes as materials for forming a light-emitting layer [Appl. Phys. Lett. 51, 913, 1987].

An organic electroluminescent device is a device changing electrical energy to light by applying electricity to an organic electroluminescent material, and generally has a structure comprising an anode, a cathode, and an organic layer between the anode and the cathode. The organic layer of an organic EL device may be comprised of a hole injection layer, a hole transport layer, an electron blocking layer, a light-emitting layer (which comprises host and dopant materials), an electron buffer layer, a hole blocking layer, an electron transport layer, an electron injection layer, etc., and the materials used for the organic layer are categorized by their functions in hole injection material, hole transport material, electron blocking material, light-emitting material, electron buffer material, hole blocking material, electron transport material, electron injection material, etc. In the organic EL device, due to an application of a voltage, holes are injected from the anode to the light-emitting layer, electrons are injected from the cathode to the light-emitting layer, and excitons of high energies are formed by a recombination of the holes and the electrons. By this energy, organic luminescent compounds reach an excited state, and light emission occurs by emitting light from energy due to the excited state of the organic luminescent compounds returning to a ground state.

The most important factor determining luminous efficiency in an organic EL device is light-emitting materials. A light-emitting material must have high quantum efficiency, high electron and hole mobility, and the formed light-emitting material layer must be uniform and stable. Light-emitting materials are categorized into blue, green, and red light-emitting materials dependent on the color of the light emission, and additionally yellow or orange light-emitting materials. In addition, light-emitting materials can also be categorized into host and dopant materials according to their functions. Recently, the development of an organic EL device providing high efficiency and long lifespan is an urgent issue. In particular, considering EL characteristic requirements for a middle or large-sized panel of OLED, materials showing better characteristics than conventional ones must be urgently developed. The host material, which acts as a solvent in a solid state and transfers energy, needs to have high purity and a molecular weight appropriate for vacuum deposition. Furthermore, the host material needs to have high glass transition temperature and high thermal degradation temperature to achieve thermal stability, high electro-chemical stability to achieve long lifespan, ease of forming an amorphous thin film, good adhesion to materials of adjacent layers, and non-migration to other layers.

Until now, fluorescent host materials have been widely used as a host material. For a blue fluorescent host material, a blue light-emitting material system of Idemitsu Kosan using 4,4'-bis(2,2'-diphenylvinyl)-1,1'-biphenyl (DPVBi), and a blue light-emitting material system of Eastman Kodak using dinaphthylanthracene and tetra(t-butyl)perylene, etc., are known. However, much research has been conducted until now in order to develop a blue fluorescent host material having improved characteristics in a device.

Korean Patent Application Laying-Open No. 2008-0047210 discloses a compound in which an aryl group is bonded to a benzocarbazole or dibenzocarbazole structure and an organic EL device comprising the same. Korean Patent Application Laying-Open No. 2013-0130747 discloses an aromatic heterocyclic ring derivative consisting of two or more carbazole derivative residual groups and a nitrogen-containing aromatic heterocyclic ring and an organic EL device comprising the same. In addition, Korean Patent Application Laying-Open No. 2010-0015581 discloses an organic electroluminescent compound in which a nitrogen-containing aromatic hydrocarbon group or an aromatic heterocyclic group is bonded to a pyrimidine ring or a triazine ring and an organic EL device comprising the same. However, necessity of compounds having superior luminous efficiency and lifespan characteristic to the compounds disclosed in the references above is continuously to the fore. WO2013/105747 (A) discloses a novel compound capable of improving the light-emitting efficiency, the stability, and the longevity of an element, an organic electronic element using same, and an electronic device thereof. EP3129446 (A) (Article 54(3) EPC document) discloses an organic electroluminescent device comprising at least one light-emitting layer between an anode and a cathode, wherein the light-emitting layer comprises a host and a phosphorescent dopant; the host consists of multi-component host compounds; at least a first host compound of the multi-component host compounds is a specific bicarbazole derivative containing an aryl group, and a second host compound is a specific carbazole derivative including a nitrogen-containing heteroaryl group. WO2010/114264 (A) discloses carbazole based compound for organic electroluminescence devices.

Accordingly, after working on obtaining a compound satisfactory to the necessity above, the present inventors found that a compound of a specific structure in which a dibenzocarbazole and a diaryltriazine are bonded via an aryl group more suitable for producing an organic EL device improved efficiency and lifespan compared to conventional organic electroluminescent compounds.

### Disclosure of the Invention

### Problems to be Solved

The objective of the present invention is to provide an organic electroluminescent device having excellent efficiency and improved lifespan.

### Solution to Problems

The invention is set out in accordance with the appended claims. The present inventors found that the above objective can be achieved by using an organic electroluminescent compound represented by the following formula 1: 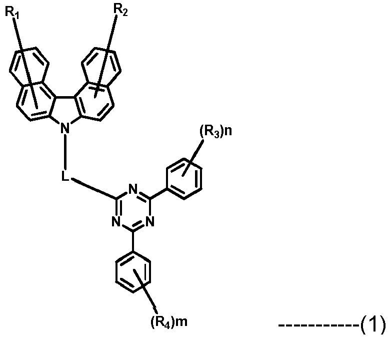 wherein
L represents a substituted or unsubstituted (C6-C30)arylene;
R₁ to R₂ each independently represent hydrogen, deuterium, a halogen, a cyano, a substituted or unsubstituted (C1-C30)alkyl, a substituted or unsubstituted (C6-C30)aryl, a substituted or unsubstituted 3- to 30-membered heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di- (C1-C30)alkylamino, a substituted or unsubstituted mono- or di- (C6-C30)arylamino, or a substituted or unsubstituted (C1-C30)alkyl(C6-C30)arylamino;
R₃ to R₄ each independently represent hydrogen, deuterium, a halogen, a cyano, a substituted or unsubstituted (C1-C30)alkyl, a substituted or unsubstituted (C6-C30)aryl, a substituted or unsubstituted 3- to 30-membered heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di- (C1-C30)alkylamino, a substituted or unsubstituted mono- or di- (C6-C30)arylamino, or a substituted or unsubstituted (C1-C30)alkyl(C6-C30)arylamino; or are linked to an adjacent substituent(s) to form a substituted or unsubstituted, mono- or polycyclic, (C3-C30) alicyclic or aromatic ring, whose carbon atom(s) may be replaced with at least one hetero atom selected from nitrogen, oxygen, and sulfur;
the heteroaryl contains at least one hetero atom selected from B, N, O, S, Si, and P; and
n and m represent an integer of 0 to 3.

### Effects of the Invention

By using the organic electroluminescent compound of the present invention as a host of the light-emitting layer, efficiency and lifespan are significantly improved compared to the conventional organic electroluminescent compounds. Specifically, by maintaining high efficiency and having significantly improved lifespan even at high luminance, the organic electroluminescent compound of the present invention shows more suitable characteristics in recent trends requiring increasing demands for high resolution.

### Embodiments of the Invention

Hereinafter, the present invention will be described in detail. However, the following description is intended to explain the invention, and is not meant in any way to restrict the scope of the invention.

The present invention relates to an organic electroluminescence device comprising an organic electroluminescent compound of formula 1.

In formula 1 above, preferably, L represents a substituted or unsubstituted (C6-C20)arylene, and R₁ to R₄ each independently represent hydrogen, or a substituted or unsubstituted (C6-C20)aryl, or R₃ to R₄ are linked to an adjacent substituent(s) to form a substituted or unsubstituted, mono- or polycyclic, (C6-C20) alicyclic or aromatic ring, whose carbon atom(s) may be replaced with at least one hetero atom selected from nitrogen, oxygen, and sulfur.

In formula 1 above, more preferably, L represents a (C6-C12)arylene unsubstituted or substituted with a (C1-C6)alkyl or a (C6-C12)aryl, and R₁ to R₄ each independently represent hydrogen or an unsubstituted (C6-C12)aryl, R₃ to R₄ are linked to an adjacent substituent(s) to form a mono- or polycyclic, (C6-C15) alicyclic or aromatic ring unsubstituted or substituted with a (C1-C6)alkyl or a (C6-C12)aryl, whose carbon atom(s) may be replaced with at least one hetero atom selected from nitrogen, oxygen, and sulfur.

Herein, "(C1-C30)alkyl" is meant to be a linear or branched alkyl having 1 to 30 carbon atoms constituting the chain, in which the number of carbon atoms is preferably 1 to 20, more preferably 1 to 10, and includes methyl, ethyl, n-propyl, isopropyl, n-butyl, isobutyl, tert-butyl, etc.; "(C2-C30)alkenyl" is meant to be a linear or branched alkenyl having 2 to 30 carbon atoms constituting the chain, in which the number of carbon atoms is preferably 2 to 20, more preferably 2 to 10, and includes vinyl, 1-propenyl, 2-propenyl, 1-butenyl, 2-butenyl, 3-butenyl, 2-methylbut-2-enyl, etc.; "(C2-C30)alkynyl" is meant to be a linear or branched alkynyl having 2 to 30 carbon atoms constituting the chain, in which the number of carbon atoms is preferably 2 to 20, more preferably 2 to 10, and includes ethynyl, 1-propynyl, 2-propynyl, 1-butynyl, 2-butynyl, 3-butynyl, 1-methylpent-2-ynyl, etc.; "(C1-C30)alkoxy" is meant to be a linear or branched alkoxy having 1 to 30 carbon atoms constituting the chain, in which the number of carbon atoms is preferably 1 to 20, more preferably 1 to 10, and includes methoxy, ethoxy, propoxy, isopropoxy, 1-ethylpropoxy, etc.; "(C3-C30)cycloalkyl" is a mono- or polycyclic hydrocarbon having 3 to 30 ring backbone carbon atoms, in which the number of carbon atoms is preferably 3 to 20, more preferably 3 to 7, and includes cyclopropyl, cyclobutyl, cyclopentyl, cyclohexyl, etc.; "5- to 7- membered heterocycloalkyl" is a cycloalkyl having 5 to 7 ring backbone atoms, including at least one heteroatom selected from B, N, O, S, Si, and P, preferably O, S, and N, and includes pyrrolidine, thiolan, tetrahydropyran, etc.; "(C6-C30)aryl(ene)" is a monocyclic or fused ring derived from an aromatic hydrocarbon having 6 to 30 ring backbone carbon atoms, in which the number of carbon atoms is preferably 6 to 20, more preferably 6 to 15, and includes phenyl, biphenyl, terphenyl, naphthyl, fluorenyl, phenanthrenyl, anthracenyl, indenyl, triphenylenyl, pyrenyl, tetracenyl, perylenyl, chrysenyl, naphthacenyl, fluoranthenyl, etc.; "3- to 30-membered heteroaryl(ene)" is an aryl having 3 to 30 ring backbone atoms, in which the number of atoms is preferably 3 to 20, more preferably 3 to 15, including at least one, preferably 1 to 4 heteroatoms selected from the group consisting of B, N, O, S, Si, and P; is a monocyclic ring, or a fused ring condensed with at least one benzene ring; may be partially saturated; may be one formed by linking at least one heteroaryl or aryl group to a heteroaryl group via a single bond(s); and includes a monocyclic ring-type heteroaryl including furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl, etc., and a fused ring-type heteroaryl including benzofuranyl, benzothiophenyl, isobenzofuranyl, dibenzofuranyl, dibenzothiophenyl, benzoimidazolyl, benzothiazolyl, benzoisothiazolyl, benzoisoxazolyl, benzoxazolyl, isoindolyl, indolyl, indazolyl, benzothiadiazolyl, quinolyl, isoquinolyl, cinnolinyl, quinazolinyl, quinoxalinyl, carbazolyl, phenoxazinyl, phenanthridinyl, benzodioxolyl, etc. Further, "halogen" includes F, Cl, Br, and I.

Herein, "substituted" in the expression, "substituted or unsubstituted," means that a hydrogen atom in a certain functional group is replaced with another atom or group, i.e. a substituent. In the present invention, the substituents of the substituted (C1-C30)alkyl, the substituted (C6-C30)aryl, the substituted 3- to 30-membered heteroaryl, the substituted (C3-C30)cycloalkyl, the substituted (C1-C30)alkoxy, the substituted tri(C1-C30)alkylsilyl, the substituted di(C1-C30)alkyl(C6-C30)arylsilyl, the substituted (C1-C30)alkyldi(C6-C30)arylsilyl, the substituted tri(C6-C30)arylsilyl, the substituted mono- or di- (C1-C30)alkylamino, the substituted mono- or di- (C6-C30)arylamino, the substituted (C1-C30)alkyl(C6-C30)arylamino, and the substituted mono- or polycyclic, (C3-C30) alicyclic or aromatic ring in R₁ to R₄ in formula 1 each independently are at least one selected from the group consisting of deuterium, a halogen, a cyano, a carboxyl, a nitro, a hydroxyl, a (C1-C30)alkyl, a halo(C1-C30)alkyl, a (C2-C30) alkenyl, a (C2-C30) alkynyl, a (C1-C30)alkoxy, a (C1-C30)alkylthio, a (C3-C30)cycloalkyl, a (C3-C30)cycloalkenyl, a 3- to 7-membered heterocycloalkyl, a (C6-C30)aryloxy, a (C6-C30)arylthio, a 5- to 30-membered heteroaryl unsubstituted or substituted with a (C6-C30)aryl, a (C6-C30)aryl unsubstituted or substituted with a 5- to 30-membered heteroaryl, a tri(C1-C30)alkylsilyl, a tri(C6-C30)arylsilyl, a di(C1-C30)alkyl(C6-C30)arylsilyl, a (C1-C30)alkyldi(C6-C30)arylsilyl, an amino, a mono- or di-(C1-C30)alkylamino, a mono- or di- (C6-C30)arylamino, a (C1-C30)alkyl(C6-C30)arylamino, a (C1-C30)alkylcarbonyl, a (C1-C30)alkoxycarbonyl, a (C6-C30)arylcarbonyl, a di(C6-C30)arylboronyl, a di(C1-C30)alkylboronyl, a (C1-C30)alkyl(C6-C30)arylboronyl, a (C6-C30)aryl(C1-C30)alkyl, and a (C1-C30)alkyl(C6-C30)aryl.

The organic electroluminescent compound represented by formula 1 includes the following compounds, but is not limited thereto:

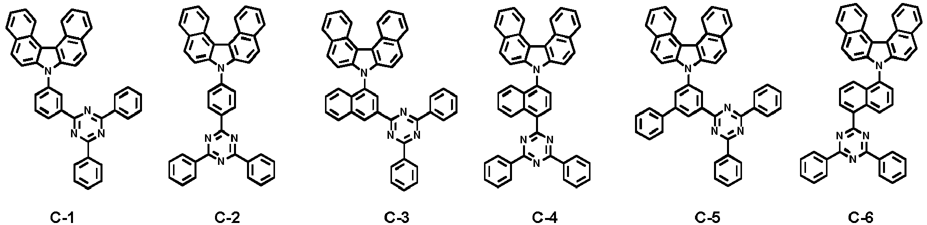

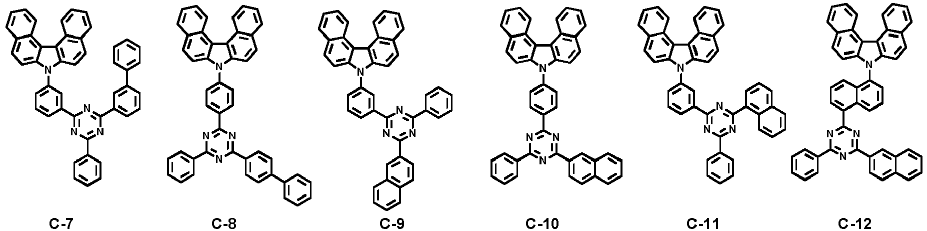

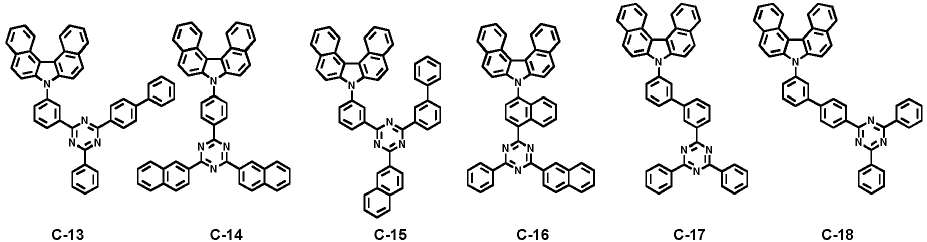

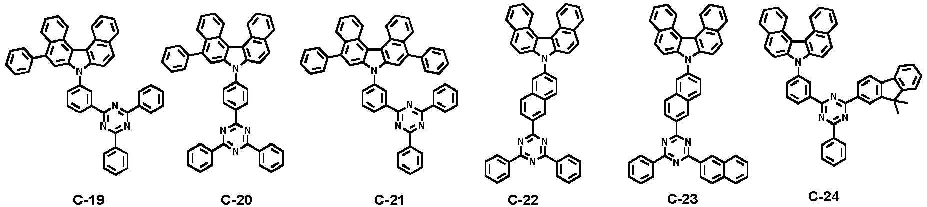

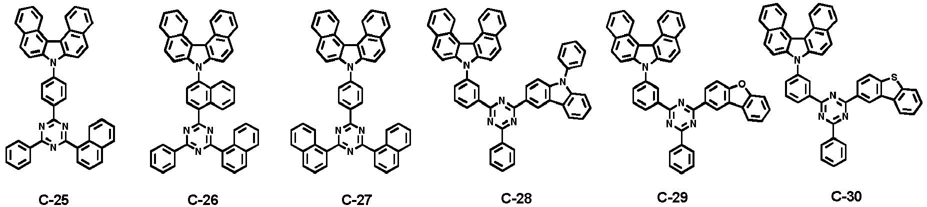

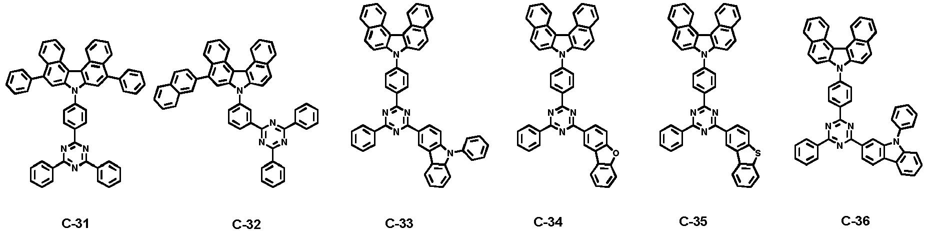

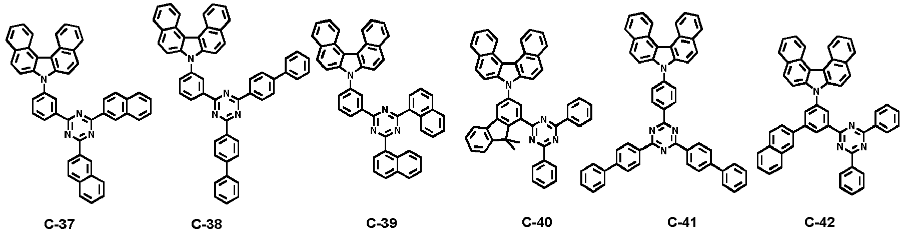

The organic electroluminescent compound of the present invention can be prepared by a synthetic method known to a person skilled in the art. For example, it can be prepared according to the following reaction scheme. 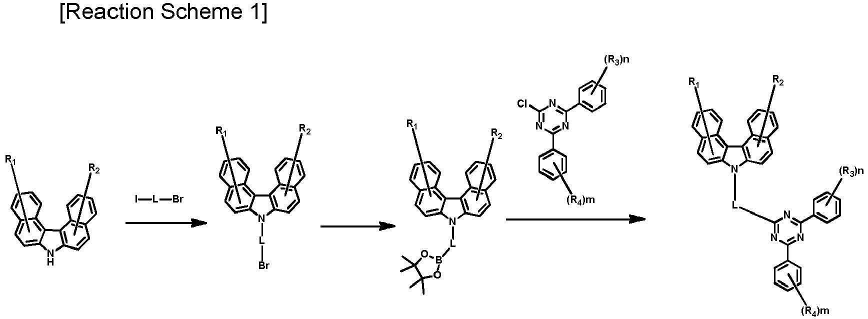 wherein L, R₁ to R₄, m, and n are as defined in formula 1.

The present invention provides an organic electroluminescent material comprising the organic electroluminescent compound of formula 1, and an organic electroluminescent device comprising the material.

The above material can be comprised of the organic electroluminescent compound according to the present invention alone, or can further include conventional materials generally used in organic electroluminescent materials.

The organic electroluminescent device comprises a first electrode; a second electrode; and at least one organic layer between the first and second electrodes. The organic layer may comprise at least one organic electroluminescent compound of formula 1.

One of the first and second electrodes can be an anode, and the other can be a cathode. The organic layer comprises a light-emitting layer, and may further comprise at least one layer selected from the group consisting of a hole injection layer, a hole transport layer, an electron transport layer, an electron buffer layer, an electron injection layer, an interlayer, a hole blocking layer, and an electron blocking layer.

The organic electroluminescent compound of formula 1 of the present invention can be comprised in the light-emitting layer as a host material, or can be comprised in the electron buffer layer. Preferably, the light-emitting layer may comprise at least one dopant. If necessary, a compound besides the organic electroluminescent compound of formula 1 can be comprised as a second host material.

Another embodiment not forming part of the claimed invention provides a material for producing an organic electroluminescent device. The material comprises the first host material and the second host material, and the first host material comprises the organic electroluminescent compound of the present invention. Herein, the weight ratio of the first host material to the second host material is in the range of 1:99 to 99:1.

The second host material can be any of the known phosphorescent hosts. Preferably, the compound may be selected from the group consisting of the compounds of formulae 2 to 6 below.

H-(Cz-L₄)ᵢ-M ---------- (2)

H-(Cz)ⱼ-L₄-M ---------- (3)

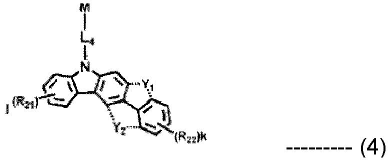

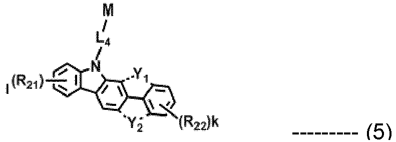

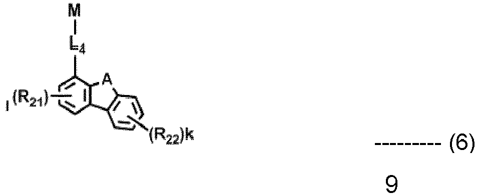

wherein Cz represents the following structure; 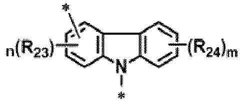 A represents -O- or -S-;
R₂₁ to R₂₄ each independently represent hydrogen, deuterium, a halogen, a substituted or unsubstituted (C1-C30)alkyl, a substituted of unsubstituted (C6-C30)aryl, a substituted or unsubstituted 5- to 30-membered heteroaryl, or -SiR₂₅R₂₆R₂₇, R₂₅ to R₂₇ each independently represent a substituted or unsubstituted (C1-C30)alkyl, or a substituted or unsubstituted (C6-C30)aryl;
L₄ represents a single bond, a substituted or unsubstituted (C6-C30)arylene, or a substituted or unsubstituted 5- to 30-membered heteroarylene;
M represents a substituted or unsubstituted (C6-C30)aryl, or a substituted or unsubstituted 5- to 30-membered heteroaryl;
Y₁ and Y₂ each independently represent -O-, -S-, -N(R₄₁)-, or -C(R₄₂)(R₄₃)-, provided that Y₁ and Y₂ do not simultaneously exist;
R₄₁ to R₄₃ each independently represent a substituted or unsubstituted (C1-C30)alkyl, a substituted or unsubstituted (C6-C30)aryl, or a substituted or unsubstituted 5- to 30-membered heteroaryl, and R₄₂ and R₄₃ may be the same or different;
i and j each independently represent an integer of 1 to 3;
k, l, m, and n each independently represent an integer of 0 to 4; and
where i, j, k, l, m, or n is an integer of 2 or more, each of (Cz-L₄), each of (Cz), each of R₂₁, each of R₂₂, each of R₂₃, or each of R₂₄ may be the same or different.

Specifically, preferable examples of the second host material are as follows: 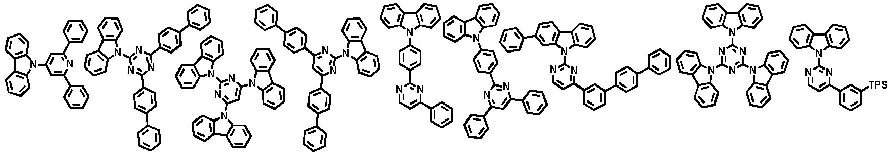 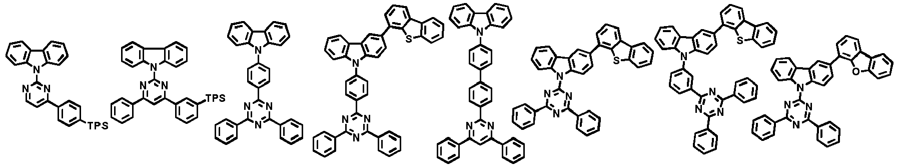 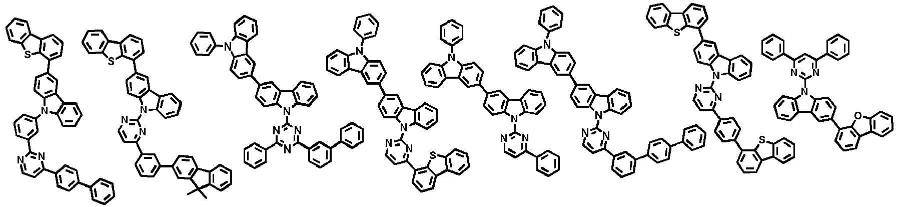 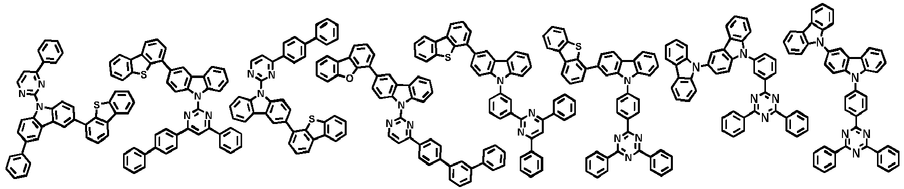 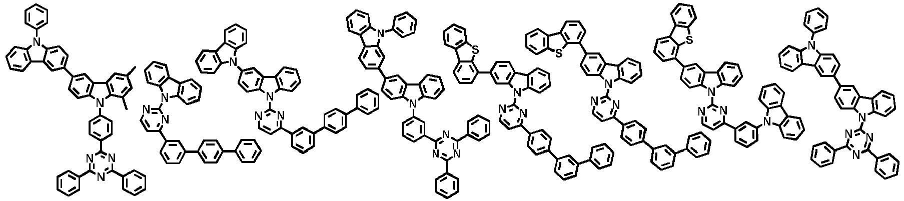 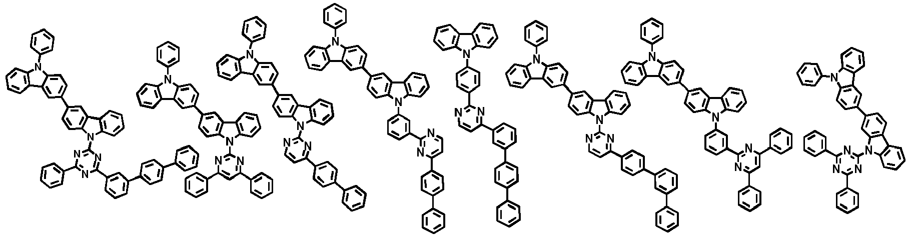 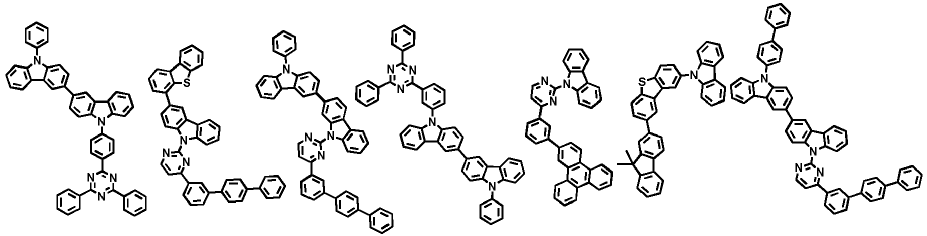 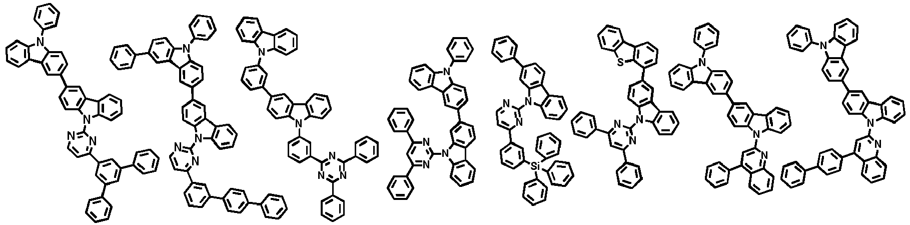 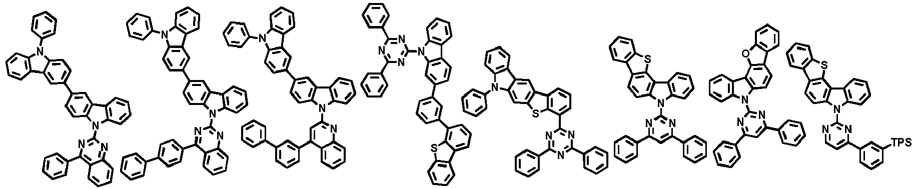 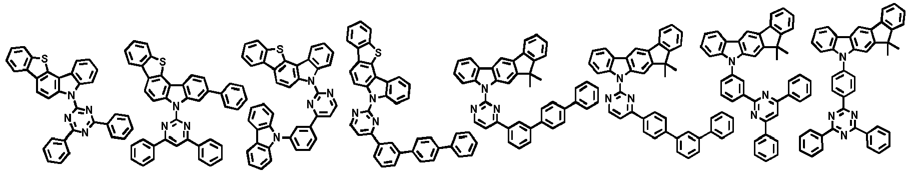 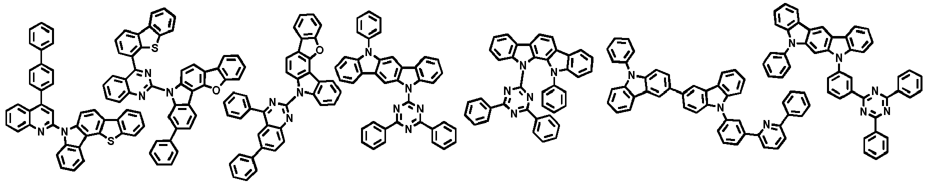 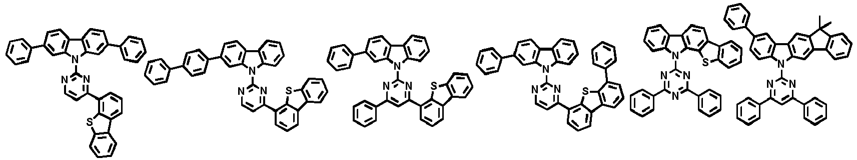 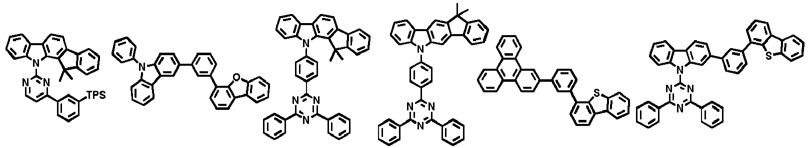 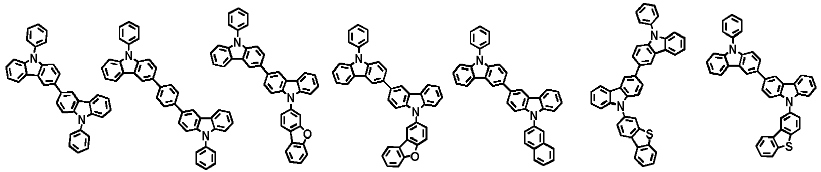 [wherein TPS represents a triphenylsilyl group]

The dopant used in the present invention is preferably at least one phosphorescent dopant. The dopant materials applied to the organic electroluminescent device according to the present invention are not limited, but may be preferably selected from metallated complex compounds of iridium, osmium, copper, and platinum, more preferably selected from ortho-metallated complex compounds of iridium, osmium, copper, and platinum, and even more preferably ortho-metallated iridium complex compounds.

The dopant comprised in the organic electroluminescent device of the present invention is preferably selected from the group consisting of the compounds of formulae 7 to 9 below. 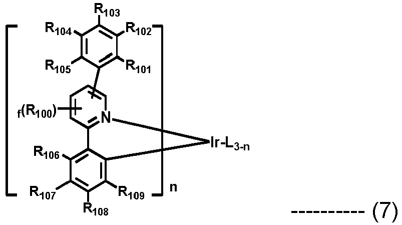 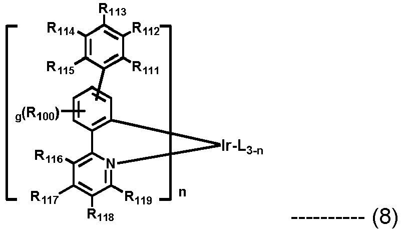 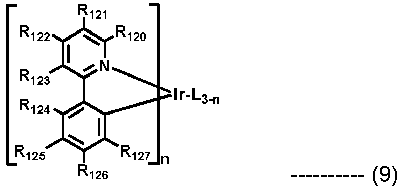 wherein L is selected from the following structures: 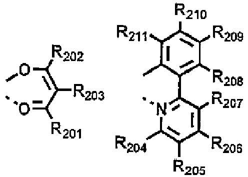 R₁₀₀ represents hydrogen, a substituted or unsubstituted (C1-C30)alkyl, or a substituted or unsubstituted (C3-C30)cycloalkyl;
R₁₀₁ to R₁₀₉, and R₁₁₁ to R₁₂₃ each independently represent hydrogen, deuterium, a halogen, a (C1-C30)alkyl unsubstituted or substituted with a halogen(s), a substituted or unsubstituted (C3-C30)cycloalkyl, a cyano, or a substituted or unsubstituted (C1-C30)alkoxy; and adjacent substituents of R₁₂₀ to R₁₂₃ may be linked to each other to form a fused ring, e.g., quinoline;
R₁₂₄ to R₁₂₇ each independently represent hydrogen, deuterium, a halogen, a substituted or unsubstituted (C1-C30)alkyl, or a substituted or unsubstituted (C6-C30)aryl; and where R₁₂₄ to R₁₂₇ are aryls, adjacent substituents of R₁₂₄ to R₁₂₇ may be linked to each other to form a fused ring, e.g., fluorene;
R₂₀₁ to R₂₁₁ each independently represent hydrogen, deuterium, a halogen, a (C1-C30)alkyl unsubstituted or substituted with a halogen(s), a substituted or unsubstituted (C3-C30)cycloalkyl, or a substituted or unsubstituted (C6-C30)aryl;
f and g each independently represent an integer of 1 to 3; where f or g is an integer of 2 or more, each of R₁₀₀ may be the same or different; and
n represents an integer of 1 to 3.

Specifically, the phosphorescent dopant materials include the following:

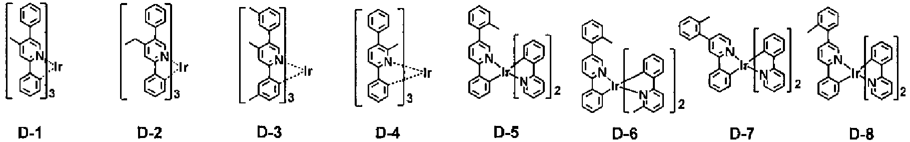

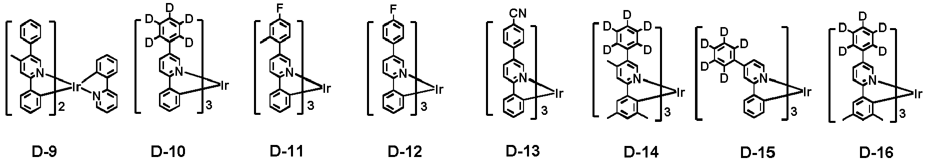

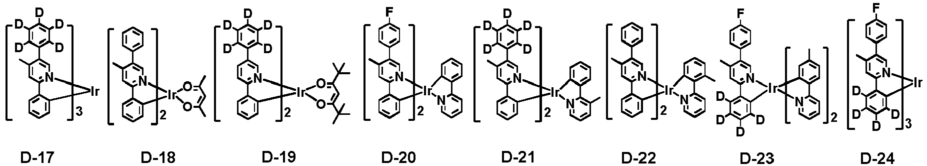

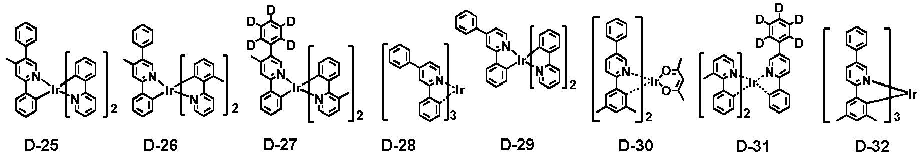

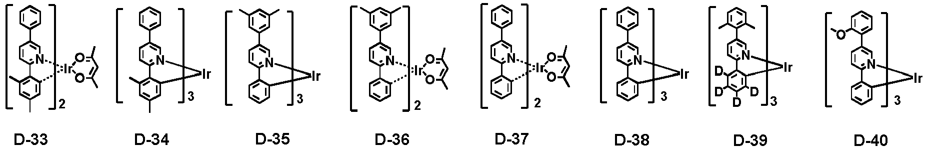

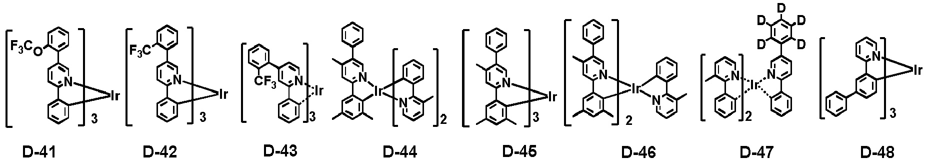

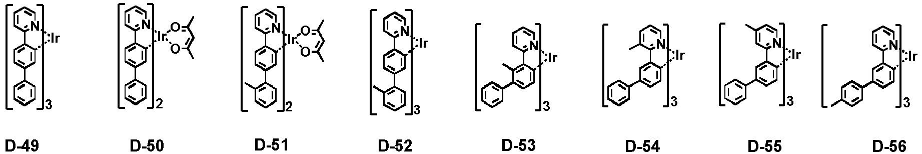

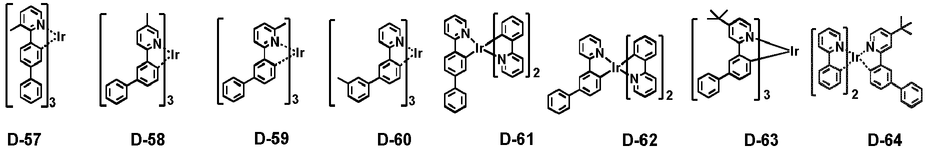

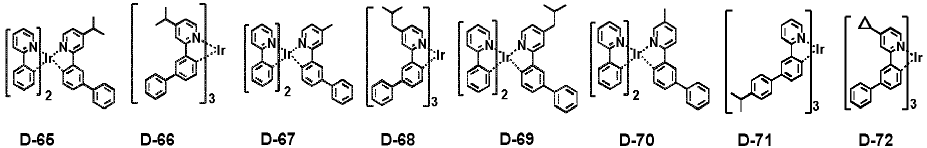

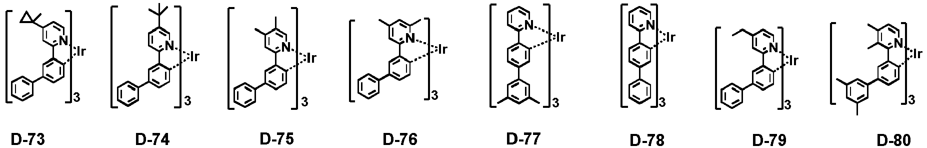

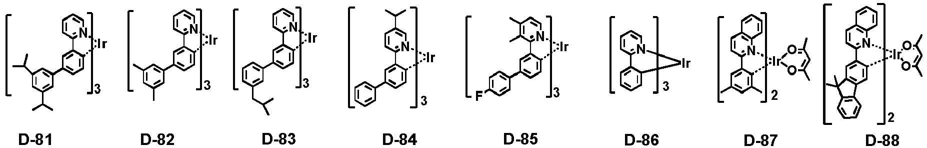

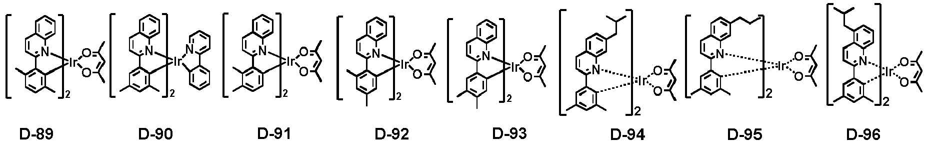

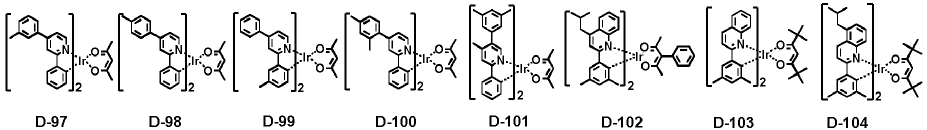

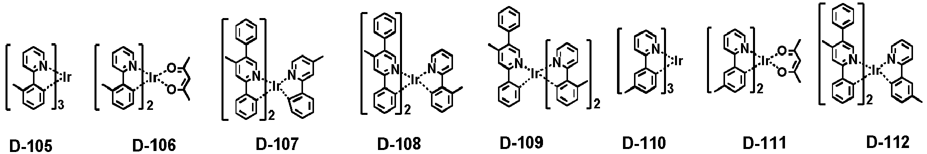

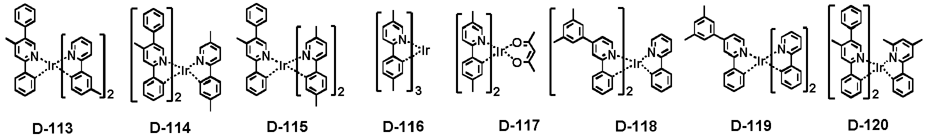

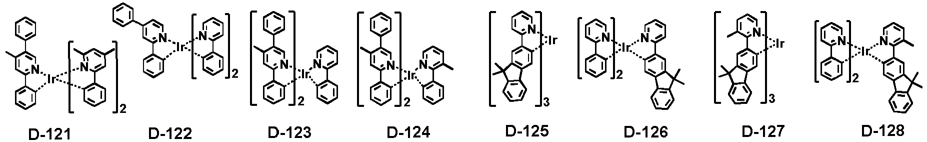

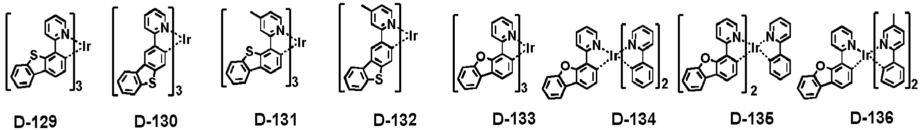

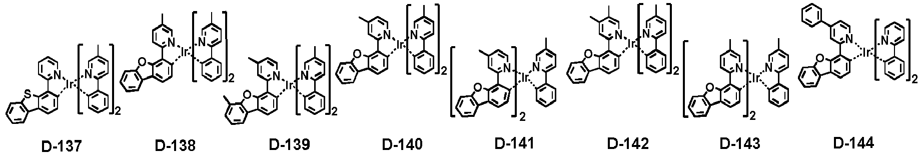

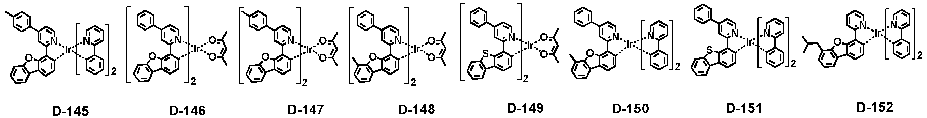

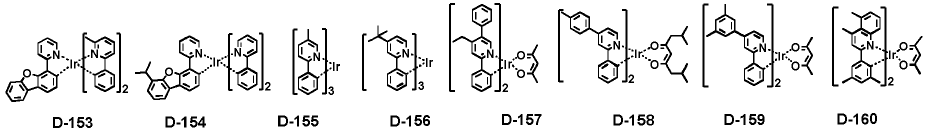

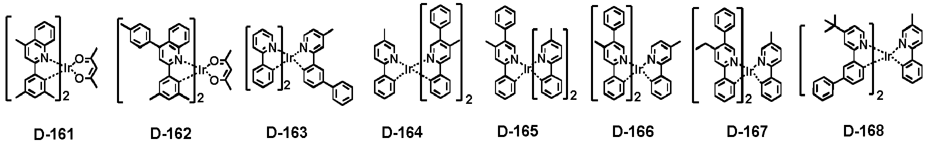

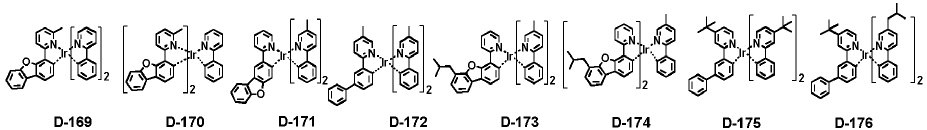

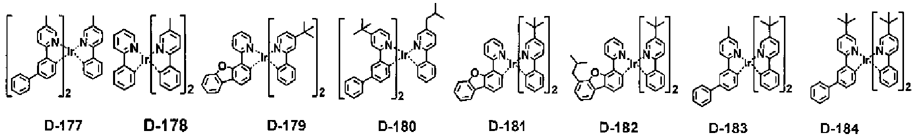

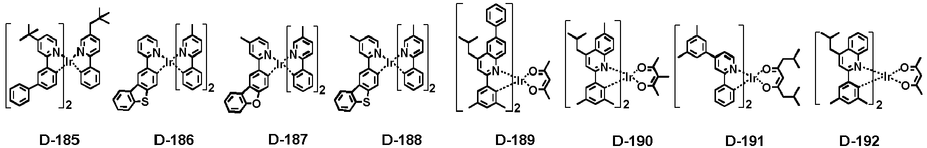

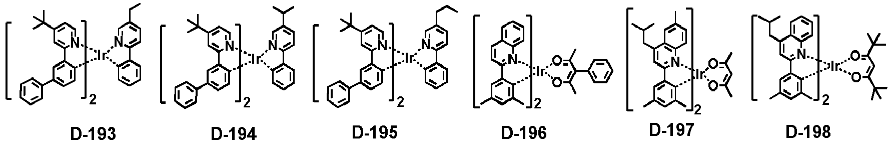

The organic electroluminescent device according to the present invention may further comprise, in addition to the organic electroluminescent compound represented by formula 1, at least one compound selected from the group consisting of arylamine-based compounds and styrylarylamine-based compounds.

In the organic electroluminescent device according to the present invention, the organic layer may further comprise at least one metal selected from the group consisting of metals of Group 1, metals of Group 2, transition metals of the 4^{th} period, transition metals of the 5^{th} period, lanthanides and organic metals of d-transition elements of the Periodic Table, or at least one complex compound comprising said metal. The organic layer may further comprise a light-emitting layer and a charge generating layer.

In addition, the organic electroluminescent device according to the present invention may emit white light by further comprising at least one light-emitting layer which comprises a blue electroluminescent compound, a red electroluminescent compound or a green electroluminescent compound known in the field, besides the organic electroluminescent compound according to the present invention. Also, if necessary, a yellow or orange light-emitting layer can be comprised in the device.

According to the present invention, at least one layer (hereinafter, "a surface layer") is preferably placed on an inner surface(s) of one or both electrode(s); selected from a chalcogenide layer, a metal halide layer, and a metal oxide layer. Specifically, a chalcogenide (including oxides) layer of silicon or aluminum is preferably placed on an anode surface of an electroluminescent medium layer, and a metal halide layer or a metal oxide layer is preferably placed on a cathode surface of an electroluminescent medium layer. Such a surface layer provides operation stability for the organic electroluminescent device. Preferably, said chalcogenide includes SiOₓ(1≤X≤2), AlOₓ(1≤X≤1.5), SiON, SiAlON, etc.; said metal halide includes LiF, MgF₂, CaF₂, a rare earth metal fluoride, etc.; and said metal oxide includes Cs₂O, Li₂O, MgO, SrO, BaO, CaO, etc.

In the organic electroluminescent device according to the present invention, a mixed region of an electron transport compound and reductive dopant, or a mixed region of a hole transport compound and an oxidative dopant is preferably placed on at least one surface of a pair of electrodes. In this case, the electron transport compound is reduced to an anion, and thus it becomes easier to inject and transport electrons from the mixed region to an electroluminescent medium. Further, the hole transport compound is oxidized to a cation, and thus it becomes easier to inject and transport holes from the mixed region to the electroluminescent medium. Preferably, the oxidative dopant includes various Lewis acids and acceptor compounds; and the reductive dopant includes alkali metals, alkali metal compounds, alkaline earth metals, rare-earth metals, and mixtures thereof. A reductive dopant layer may be employed as a charge generating layer to prepare an electroluminescent device having two or more electroluminescent layers and emitting white light.

In order to form each layer of the organic electroluminescent device according to the present invention, dry film-forming methods such as vacuum evaporation, sputtering, plasma and ion plating methods, or wet film-forming methods such as spin coating, dip coating, and flow coating methods can be used.

When using a wet film-forming method, a thin film can be formed by dissolving or diffusing materials forming each layer into any suitable solvent such as ethanol, chloroform, tetrahydrofuran, dioxane, etc. The solvent can be any solvent where the materials forming each layer can be dissolved or diffused, and where there are no problems in film-formation capability.

Hereinafter, the organic electroluminescent compound, the preparation method of the compound, and the properties of the device comprising the organic electroluminescent compound will be explained in detail with reference to the following examples.

### Example 1: Preparation of compound C-1

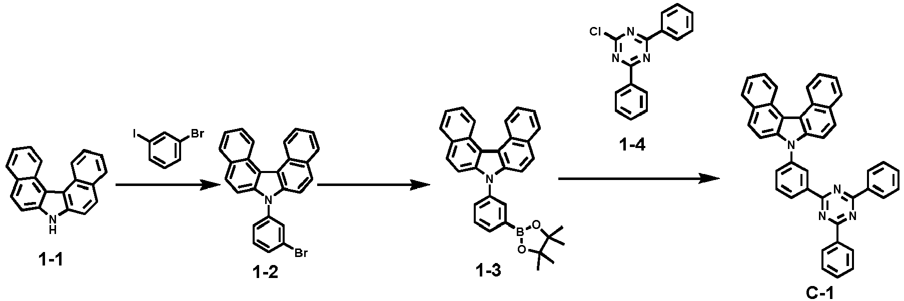

### Preparation of compound 1-2

After dissolving compound 1-1 (7H-benzo[c]carbazole, 17.6 g, 65.8 mmol), 3-iodo-1-bromobenzene (23 g, 78.9 mmol), Cul (6.3 g, 33 mmol), ethylenediamine (EDA) (9mL, 130 mmol), and K₃PO₄ (42 g, 197.4 mmol) in toluene 500 mL in a flask, the mixture was refluxed at 120°C for 5 hours. After completing the reaction, an organic layer was extracted with ethyl acetate (EA), residual moisture was removed using magnesium sulfate and dried, and separated with column chromatography to obtain compound 1-2 (20 g, yield: 71%).

### Preparation of compound 1-3

After dissolving compound 1-2 (15 g, 35.3 mmol), 4,4,4',4',5,5,5',5'-octamethyl-2,2'-bi(1,3,2-dioxaborolane) (14 g, 53 mmol), potassium acetate (KOAc) (11 g, 106 mmol), and bis(triphenylphosphine)palladium(II)dichloride (Pd(PPh₃)₂Cl₂) (2.5 g, 0.0035 mmol) in dioxane 300 mL in a flask, the mixture was refluxed. After completing the reaction, an organic layer was extracted with EA, residual moisture was removed using magnesium sulfate and dried, and separated with column chromatography to obtain compound 1-3 (10 g, yield: 61%).

### Preparation of compound C-1

After dissolving compound 1-3 (10 g, 21.7 mmol), compound 1-4 (12 g, 0.043 mmol), tetrakis(triphenylphosphine)palladium (Pd(PPh₃)₄) (1.3 g, 1.0 mmol), and K₂CO₃ (8.8 g, 65 mmol) in a mixture solvent of toluene, ethanol (EtOH), and H₂O in a flask, the mixture was refluxed at 120°C for one day. After completing the reaction, an organic layer was extracted with EA, dried, and separated with column chromatography to obtain compound C-1 (7.8 g, yield: 48%).

| | MW | UV | PL | M.P. |
|---|---|---|---|---|
| C-1 | 574.67 | 334 nm | 482 nm | 292°C |

### Example 2: Preparation of compound C-2

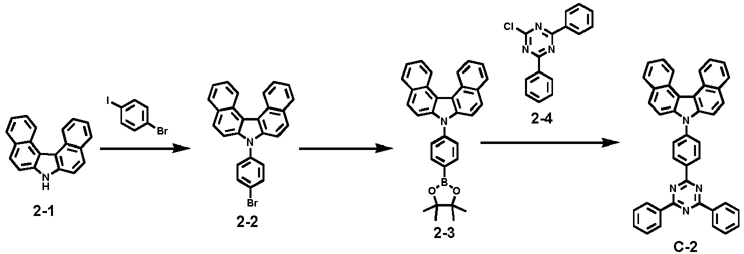

### Preparation of compound 2-2

After dissolving compound 2-1 (7H-benzo[c]carbazole, 17.6 g, 65.8 mmol), 4-iodo-1-bromobenzene (23 g, 78.9 mmol), Cul (6.3 g, 33 mmol), EDA (9mL, 130 mmol), and K₃PO₄ (42 g, 197.4 mmol) in toluene 500 mL in a flask, the mixture was refluxed at 120°C for 5 hours. After completing the reaction, an organic layer was extracted with EA, residual moisture was removed using magnesium sulfate and dried, and separated with column chromatography to obtain compound 2-2 (20 g, yield: 71%).

### Preparation of compound 2-3

After dissolving compound 2-2 (15 g, 35.3 mmol), 4,4,4',4',5,5,5',5'-octamethyl-2,2'-bi(1,3,2-dioxaborolane) (14 g, 53 mmol), KOAc (11 g, 106 mmol), and Pd(PPh₃)₂Cl₂ (2.5 g, 0.0035 mmol) in dioxane 300 mL in a flask, the mixture was refluxed. After completing the reaction, an organic layer was extracted with EA, residual moisture was removed using magnesium sulfate and dried, and separated with column chromatography to obtain compound 2-3 (10 g, yield: 61%).

### Preparation of compound C-2

After dissolving compound 2-3 (10 g, 21.7 mmol), compound 2-4 (12 g, 0.043 mmol), Pd(PPh₃)₄ (1.3 g, 1.0 mmol), and K₂CO₃ (8.8 g, 65 mmol) in a mixture solvent of toluene, ethanol, and H₂O in a flask, the mixture was refluxed at 120°C for one day. After completing the reaction, an organic layer was extracted with EA, dried, and separated with column chromatography to obtain compound C-2 (3.7 g, yield: 88%).

| | MW | UV | PL | M.P. |
|---|---|---|---|---|
| C-2 | 574.67 | 344 nm | 473 nm | 323°C |

### Example 3: Preparation of compound C-17

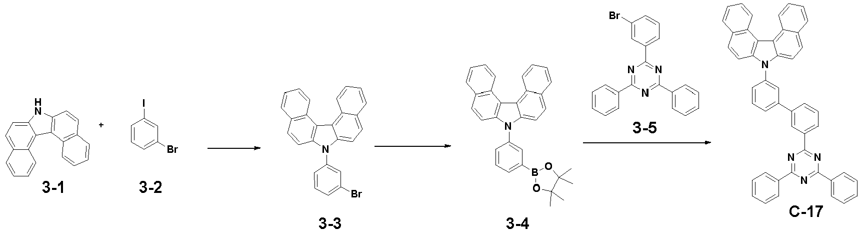

### Preparation of compound 3-3

After introducing 7H-benzo[c,g]carbazole (20 g, 74.80 mmol), 1-bromo-3-iodobenzene (23.7 mL, 187.00 mmol), Cul (7.1 g, 37.4 mmol), K₃PO₄ (39.7 g, 187.00 mmol), and toluene 380 mL in a flask, the mixture was refluxed at 120°C for 2 hours. After completing the reaction, the mixture was filtered with dichloromethane (DCM), dried, and separated with column chromatography to obtain compound 3-3 (23.6 g, yield: 75%).

### Preparation of compound 3-4

After dissolving compound 3-3 (70 g, 165.80 mmol), pinacolato diboron (51 g, 198.9 mmol), PdCl₂(PPh₃)₂ (5.8 g, 8.29 mmol), and KOAc (33 g, 331.60 mmol) in 1,4-dioxane 830 mL in a flask, the mixture was stirred under reflux for one day. The mixture was then extracted with EA and separated with column chromatography to obtain compound 3-4 (69 g, yield: 88%).

### Preparation of compound C-17

After dissolving compound 3-4 (7.0 g, 14.90 mmol), compound 3-5 (7.0 g, 17.90 mmol), Pd(PPh₃)₄ (860 mg, 0.75 mmol), and K₂CO₃ (6.2 g, 44.70 mmol) in a mixture solvent of toluene 75 mL, EtOH 20 mL, and H₂O 20 mL in a flask, the mixture was stirred under reflux for 1 hour. After cooling the mixture to room temperature, methanol (MeOH) was added thereto, a solid was filtered and recrystallized with toluene, and separated with column chromatography to obtain compound C-17 (4.6 g, yield: 47%).

| | MW | UV | PL | M.P. |
|---|---|---|---|---|
| C-17 | 650.77 | 378 nm | 393 nm | 267°C |

### Example 4: Preparation of compound C-9

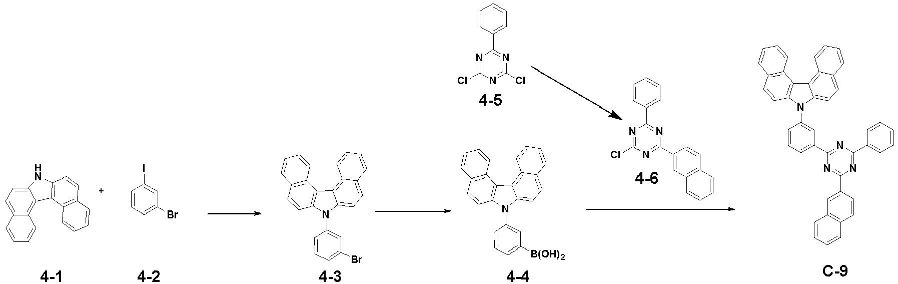

### Preparation of compound 4-3

After introducing 7H-dibenzo[c,g]carbazole (20 g, 74.80 mmol), 1-bromo-3-iodobenzene (23.7 mL, 187.00 mmol), Cul (7.1 g, 37.4 mmol), K₃PO₄ (39.7 g, 187.00 mmol), and toluene 380 mL in a flask and dissolving the solutes in the solvent, the mixture was refluxed at 120°C for 2 hours. After completing the reaction, the mixture was filtered with DCM, dried, and separated with column chromatography to obtain compound 4-3 (23.6 g, yield: 75%).

### Preparation of compound 4-4

After dissolving compound 4-3 (24.0 g, 55.90 mmol) and n-BuLi (34 mL, 83.80 mmol) in tetrahydrofuran (THF) 280 mL in a flask, the mixture was stirred at -78°C for one hour. Trimethyl borate (B(OMe)₃) (9.4 mL, 83.80 mmol) was then added dropwise to the mixture, and the mixture was stirred at room temperature for 4 hours. The mixture was then extracted with EA, and a solid was filtered with hexane (Hex) to obtain compound 4-4 (15.9 g, yield: 74%).

### Preparation of compound 4-6

After introducing compound 4-5 (20 g, 88.5 mmol), 2-naphthyl boronic acid (16.7 g, 97.3 mmol), PdCl₂(PPh₃)₂ (2.0 g, 2.92 mmol), Na₂CO₃ (23.5 g, 221.3 mmol), THF 440 mL, and H₂O 150 mL in a flask, the mixture was stirred for 2 hours at 65°C. After cooling the mixture to room temperature, distilled water was added thereto. After completing the reaction, an organic layer was extracted with EA, residual moisture was removed using magnesium sulfate and dried, and separated with column chromatography to obtain compound 4-6 (21 g, yield: 81%).

### Preparation of compound C-9

After dissolving compound 4-4 (4.0 g, 10.30 mmol), compound 4-6 (4.9 g, 15.50 mmol), Pd(PPh₃)₄ (600 mg, 0.52 mmol), and K₂CO₃ (4.3 g, 30.90 mmol) in a mixture solvent of toluene 50 mL, EtOH 12 mL, and H₂O 12 mL in a flask, the mixture was stirred under reflux for 4 hours. After cooling the mixture to room temperature, MeOH was added thereto, a solid was filtered, and separated with column chromatography to obtain compound C-9 (5.6 g, yield: 67%).

| | MW | UV | PL | M.P. |
|---|---|---|---|---|
| C-9 | 624.73 | 376 nm | 481 nm | 200.6°C |

### Example 5: Preparation of compound C-7

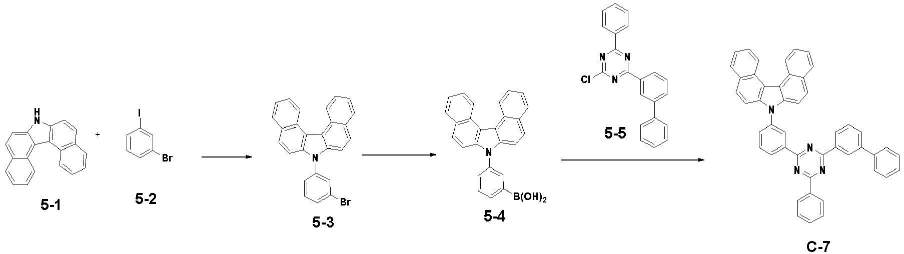

### Preparation of compound 5-3

After introducing 7H-dibenzo[c,g]carbazole (20 g, 74.80 mmol), 1-bromo-3-iodobenzene (23.7 mL, 187.00 mmol), Cul (7.1 g, 37.4 mmol), K₃PO₄ (39.7 g, 187.00 mmol), and toluene 380 mL in a flask and dissolving the solutes in the solvent, the mixture was refluxed at 120°C for 2 hours. After completing the reaction, the mixture was filtered with DCM, dried, and separated with column chromatography to obtain compound 5-3 (23.6 g, yield: 75%).

### Preparation of compound 5-4

After dissolving compound 5-3 (24.0 g, 55.90 mmol) and n-BuLi (34 mL, 83.80 mmol) in THF 280 mL in a flask, the mixture was stirred at -78°C for one hour. B(OMe)₃ (9.4 mL, 83.80 mmol) was then added dropwise to the mixture, and the mixture was stirred at room temperature for 4 hours. The mixture was then extracted with EA, and a solid was filtered with Hex to obtain compound 5-4 (15.9 g, yield: 74%).

### Preparation of compound C-7

After dissolving compound 5-4 (5.0 g, 12.90 mmol), compound 5-5 (6.7 g, 19.40 mmol), Pd(PPh₃)₄ (745 mg, 0.66 mmol), and K₂CO₃ (5.3 g, 38.70 mmol) in a mixture solvent of toluene 65 mL, EtOH 16 mL, and H₂O 16 mL in a flask, the mixture was stirred under reflux for 1 hour. After cooling the mixture to room temperature, the mixture was extracted with EA, and separated with column chromatography to obtain compound C-7 (3.8 g, yield: 45%).

| | MW | UV | PL | M.P. |
|---|---|---|---|---|
| C-7 | 650.77 | 324 nm | 393 nm | 146.6°C |

### Example 6: Preparation of compound C-37

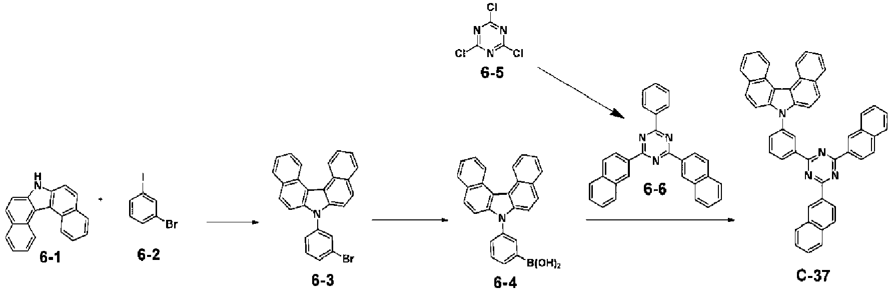

### Preparation of compound 6-3

After introducing 7H-dibenzo[c,g]carbazole (20 g, 74.80 mmol), 1-bromo-3-iodobenzene (23.7 mL, 187.00 mmol), Cul (7.1 g, 37.4 mmol), K₃PO₄ (39.7 g, 187.00 mmol), and toluene 380 mL in a flask and dissolving the solutes in the solvent, the mixture was refluxed at 120°C for 2 hours. After completing the reaction, the mixture was filtered with DCM, dried, and separated with column chromatography to obtain compound 6-3 (23.6 g, yield: 75%).

### Preparation of compound 6-4

After dissolving compound 6-3 (24.0 g, 55.90 mmol) and n-BuLi (34 mL, 83.80 mmol) in THF 280 mL in a flask, the mixture was stirred at -78°C for one hour. B(OMe)₃ (9.4 mL, 83.80 mmol) was then added dropwise to the mixture, and the mixture was stirred at room temperature for 4 hours. The mixture was then extracted with EA, and a solid was filtered with Hex to obtain compound 6-4 (15.9 g, yield: 74%).

### Preparation of compound 6-6

After dissolving compound 6-5 (20 g, 108.5 mmol), 2-naphthyl boronic acid (37.3 g, 216.9 mmol), PdCl₂(PPh₃)₂ (2.3 g, 3.3 mmol), and Na₂CO₃ (28.7 g, 271.3 mmol) in a mixture solvent of toluene 540 mL and H₂O 135 mL in a flask, the mixture was stirred for 2 hours at 95°C. After cooling the mixture to room temperature, distilled water was added thereto. After completing the reaction, an organic layer was extracted with ethyl acetate, residual moisture was removed using magnesium sulfate and dried, and separated with column chromatography to obtain compound 6-6 (9 g, yield: 21%).

### Preparation of compound C-37

After dissolving compound 6-4 (5.0 g, 12.9 mmol), compound 6-6 (5.7 g, 15.50 mmol), Pd(PPh₃)₄ (745 mg, 0.65 mmol), and K₂CO₃ (5.3 g, 38.70 mmol) in a mixture solvent of toluene 65 mL, EtOH 16 mL, and H₂O 16 mL in a flask, the mixture was stirred under reflux for 4 hours. After cooling the mixture to room temperature, MeOH was added thereto, a solid was filtered, and separated with column chromatography to obtain compound C-37 (3.7 g, yield: 43%).

| | MW | UV | PL | M.P. |
|---|---|---|---|---|
| C-37 | 674.79 | 340 nm | 489 nm | 290.5°C |

### Example 7: Preparation of compound C-8

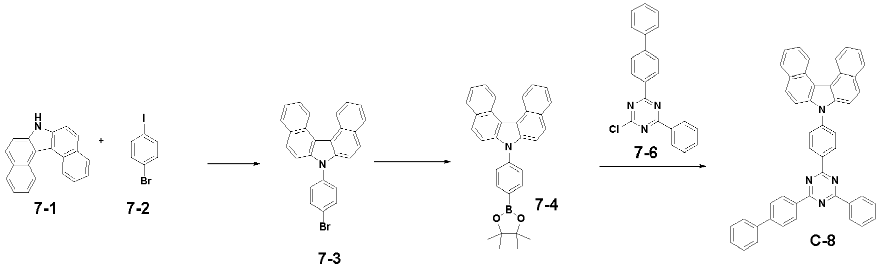

### Preparation of compound 7-3

After introducing 7H-dibenzo[c,g]carbazole (45 g, 168.0 mmol), 1-bromo-4-iodobenzene (95 g, 336.0 mmol), Cul (16 g, 84 mmol), EDA (22.5 mL, 336 mmol), K₃PO₄ (89 g, 420.00 mmol), and toluene 840 mL in a flask and dissolving the solutes in the solvent, the mixture was refluxed at 120°C for 2 hours. After completing the reaction, the mixture was filtered with DCM, dried, and separated with column chromatography to obtain compound 7-3 (41.8 g, yield: 59%).

### Preparation of compound 7-4

After dissolving compound 7-3 (7.2 g, 17.0 mmol), pinacolato diboron (5.2 g, 20.5 mmol), PdCl₂(PPh₃)₂ (1.2 g, 1.7 mmol), and KOAc (3.4 g, 34.0 mmol) in 1,4-dioxane 85 mL in a flask, the mixture was stirred under reflux for 3 hours. The mixture was then extracted with EA and separated with column chromatography to obtain compound 7-4 (6.4 g, yield: 80%).

### Preparation of compound C-8

After dissolving compound 7-4 (5.9 g, 12.60 mmol), compound 7-6 (4.3 g, 12.60 mmol), Pd(PPh₃)₄ (728 mg, 0.63 mmol), and K₂CO₃ (3.5 g, 25.20 mmol) in a mixture solvent of toluene 63 mL, EtOH 16 mL, and H₂O 16 mL in a flask, the mixture was stirred under reflux for 2 hours. After cooling the mixture to room temperature, MeOH was added thereto, a solid was filtered, and separated with column chromatography to obtain compound C-8 (4.0 g, yield: 49%).

| | MW | UV | PL | M.P. |
|---|---|---|---|---|
| C-8 | 650.77 | 392 nm | 473 nm | 225.5°C |

### Example 8: Preparation of compound C-13

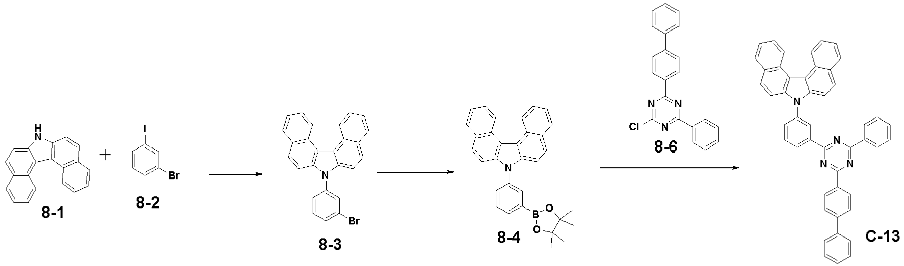

### Preparation of compound 8-3

After introducing 7H-dibenzo[c,g]carbazole (20 g, 74.80 mmol), 1-bromo-3-iodobenzene (23.7 mL, 187.00 mmol), Cul (7.1 g, 37.4 mmol), K₃PO₄ (39.7 g, 187.00 mmol), and toluene 380 mL in a flask and dissolving the solutes in the solvent, the mixture was refluxed at 120°C for 2 hours. After completing the reaction, the mixture was filtered with DCM, dried, and separated with column chromatography to obtain compound 8-3 (23.6 g, yield: 75%).

### Preparation of compound 8-4

After dissolving compound 8-3 (7.2 g, 17.0 mmol), pinacolato diboron (5.2 g, 20.5 mmol), PdCl₂(PPh₃)₂ (1.2 g, 1.7 mmol), and KOAc (3.4 g, 34.0 mmol) in 1,4-dioxane 85 mL in a flask, the mixture was stirred under reflux for 3 hours. The mixture was then extracted with EA and separated with column chromatography to obtain compound 8-4 (6.4 g, yield: 80%).

### Preparation of compound C-13

After dissolving compound 8-4 (5.5 g, 11.60 mmol), compound 8-6 (4.0 g, 11.60 mmol), Pd(PPh₃)₄ (670 mg, 0.58 mmol), and NaHCO₃ (2.0 g, 23.20 mmol) in a mixture solvent of THF 63 mL and H₂O 23 mL in a flask, the mixture was stirred under reflux for 2 hours. After cooling the mixture to room temperature, MeOH was added thereto, a solid was filtered, and separated with column chromatography to obtain compound C-13 (4.0 g, yield: 57%).

| | MW | UV | PL | M.P. |
|---|---|---|---|---|
| C-13 | 650.77 | 382 nm | 485 nm | 242.2°C |

### Example 9: Preparation of compound C-4

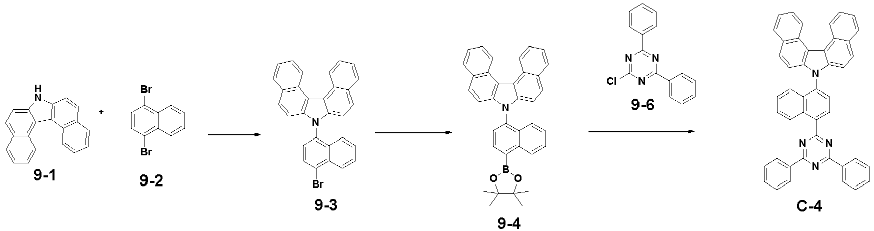

### Preparation of compound 9-3

After introducing 7H-dibenzo[c,g]carbazole (20 g, 74.80 mmol), 1,4-dibromonaphthalene (53.5 g, 187.00 mmol), CuSO₄·5H₂O (1.9 g, 7.5 mmol), K₂CO₃ (20.7 g, 149.60 mmol), and 1,2-dichlorobenzene 374 mL in a flask and dissolving the solutes in the solvent, the mixture was refluxed at 200°C for 2 days. After completing the reaction, the mixture was filtered with DCM, dried, and separated with column chromatography to obtain compound 9-3 (14.9 g, yield: 42%).

### Preparation of compound 9-4

After dissolving compound 9-3 (14.9 g, 31.5 mmol), pinacolato diboron (9.6 g, 37.9 mmol), PdCl₂(PPh₃)₂ (2.2 g, 3.2 mmol), and KOAc (6.2 g, 63.0 mmol) in 1,4-dioxane 158 mL in a flask, the mixture was stirred under reflux for 5 hours. The mixture was then extracted with MC and separated with column chromatography to obtain compound 9-4 (8.6 g, yield: 52%).

### Preparation of compound C-4

After dissolving compound 9-4 (4.0 g, 7.70 mmol), compound 9-6 (2.5 g, 9.20 mmol), Pd(PPh3)4 (445 mg, 0.39 mmol), and K₂CO₃ (2.2 g, 15.40 mmol) in a mixture solvent of toluene 40 mL, EtOH 10 mL, and H₂O 10 mL in a flask, the mixture was stirred under reflux for 4 hours. After cooling the mixture to room temperature, MeOH was added thereto, a solid was filtered, and separated with column chromatography to obtain compound C-4 (3.1 g, yield: 65%).

| | MW | UV | PL | M.P. |
|---|---|---|---|---|
| C-4 | 624.73 | 392 nm | 511 nm | 292.7°C |

### Example 10: Preparation of compound C-5

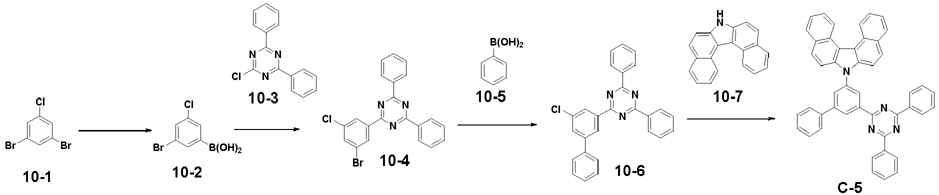

### Preparation of compound 10-2

After dissolving compound 10-1 (40.0 g, 147.96 mmol) and n-BuLi (71 mL, 177.55 mmol) in a mixture solvent of toluene 555 mL and THF 185 mL in a flask, the mixture was stirred at -78°C for one hour. B(OiPr)₃ (40.0 mL, 177.55 mmol) was then added dropwise to the mixture, and the mixture was stirred at room temperature for 3 hours. The mixture was then extracted with EA, and a solid was filtered with Hex to obtain compound 10-2 (11.5 g, yield: 33%).

### Preparation of compound 10-4

After dissolving compound 10-2 (6.7 g, 28.50 mmol), compound 10-3 (15.2 g, 57.0 mmol), Pd(PPh₃)₄ (1.6 g, 1.43 mmol), and K₂CO₃ (7.9 g, 57.00 mmol) in a mixture solvent of toluene 140 mL, EtOH 35 mL, and H₂O 35 mL in a flask, the mixture was stirred under reflux for 3 hours. After cooling the mixture to room temperature, MeOH was added thereto, a solid was filtered, and separated with column chromatography to obtain compound 10-4 (8.8 g, yield: 73%).

### Preparation of compound 10-6

After dissolving compound 10-4 (8.3 g, 19.6 mmol), phenyl boronic acid (2.9 g, 23.6 mmol), Pd(PPh₃)₄ (1.2 g, 0.98 mmol), and K₂CO₃ (5.4 g, 39.20 mmol) in a mixture solvent of toluene 100 mL, EtOH 25 mL, and H₂O 25 mL in a flask, the mixture was stirred under reflux for 4 hours. The mixture was then cooled to room temperature, extracted with EA, and separated with column chromatography to obtain compound 10-6 (6.2 g, yield: 67%).

### Preparation of compound C-5

After dissolving compound 10-6 (5.4 g, 12.86 mmol), compound 10-7 (3.8 g, 14.10 mmol), Pd₂(dba)₃ (589 mg, 0.64 mmol), 2-dicyclohexylphosphino-2',6'-dimethoxybiphenyl (S-phos) (528 mg, 1.29 mmol), and NaOtBu (1.9 g, 19.29 mmol) in o-xylene 64 mL in a flask, the mixture was stirred under reflux for 6 hours. After cooling the mixture to room temperature, MeOH was added thereto, a solid was filtered, and separated with column chromatography to obtain compound C-5 (5.7 g, yield: 68%).

| | MW | UV | PL | M.P. |
|---|---|---|---|---|
| C-5 | 650.77 | 378 nm | 485 nm | 307.7°C |

### Example 11: Preparation of compound C-16

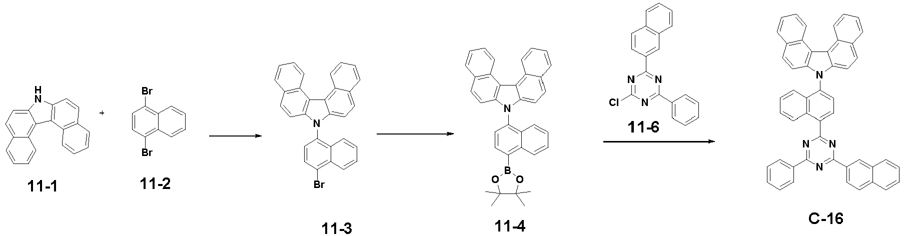

### Preparation of compound 11-3

After introducing 7H-dibenzo[c,g]carbazole (20 g, 74.80 mmol), 1,4-dibromonaphthalene (53.5 g, 187.00 mmol), CuSO₄·5H₂O (1.9 g, 7.5 mmol), K₂CO₃ (20.7 g, 149.60 mmol), and 1,2-dichlorobenzene 374 mL in a flask and dissolving the solutes in the solvent, the mixture was refluxed at 200°C for 2 days. After completing the reaction, the mixture was filtered with DCM, dried, and separated with column chromatography to obtain compound 11-3 (14.9 g, yield: 42%).

### Preparation of compound 11-4

After dissolving compound 11-3 (14.9 g, 31.5 mmol), pinacolato diboron (9.6 g, 37.9 mmol), PdCl₂(PPh₃)₂ (2.2 g, 3.2 mmol), and KOAc (6.2 g, 63.0 mmol) in 1,4-dioxane 158 mL in a flask, the mixture was stirred under reflux for 5 hours. The mixture was then extracted with MC and separated with column chromatography to obtain compound 11-4 (8.6 g, yield: 52%).

### Preparation of compound C-16

After dissolving compound 11-4 (5.7 g, 10.97 mmol), compound 11-6 (4.2 g, 13.20 mmol), Pd(PPh₃)₄ (634 mg, 0.55 mmol), and K₂CO₃ (3.0 g, 21.94 mmol) in a mixture solvent of toluene 60 mL, EtOH 15 mL, and H₂O 15 mL in a flask, the mixture was stirred under reflux for 2 hours and 30 minutes. After cooling the mixture to room temperature, MeOH was added thereto, a solid was filtered, and separated with column chromatography to obtain compound C-16 (5.1 g, yield: 69%).

| | MW | UV | PL | M.P. |
|---|---|---|---|---|
| C-16 | 674.81 | 412 nm | 585 nm | 281.9°C |

### Device Examples 1-1 to 1-12: Production of an organic electroluminescent device comprising the organic electroluminescent compound according to the present invention as a host

An OLED device was produced comprising the organic electroluminescent compound according to the present invention. A transparent electrode indium tin oxide (ITO) thin film (10 Ω/sq) on a glass substrate for an organic light-emitting diode (OLED) device (Geomatec, Japan) was subjected to an ultrasonic washing with trichloroethylene, acetone, ethanol, and distilled water, sequentially, and was then stored in isopropanol. Next, the ITO substrate was mounted on a substrate holder of a vacuum vapor depositing apparatus. N⁴,N⁴'-diphenyl-N⁴, N⁴'-bis(9-phenyl-9H-carbazol-3-yl)-[1,1'-biphenyl]-4,4'-diamine was introduced into a cell of said vacuum vapor depositing apparatus, and then the pressure in the chamber of said apparatus was controlled to 10⁻⁶ torr. Thereafter, an electric current was applied to the cell to evaporate the above introduced material, thereby forming a first hole injection layer having a thickness of 80 nm on the ITO substrate. Dipyrazino[2,3-f:2',3'-h]quinoxaline-2,3,6,7,10,11-hexacarbonitrile was then introduced into another cell of said vacuum vapor depositing apparatus, and was evaporated by applying an electric current to the cell, thereby forming a second hole injection layer having a thickness of 5 nm on the first hole injection layer. N-([1,1'-biphenyl]-4-yl)-9,9-dimethyl-N-(4-(9-phenyl-9H-carbazol-3-yl)phenyl)-9H-fluorene-2-amine was introduced into another cell of said vacuum vapor depositing apparatus, and was evaporated by applying an electric current to the cell, thereby forming a first hole transport layer having a thickness of 10 nm on the second hole injection layer. N-(4-(9,9-diphenyl-9H,9'H-[2,9'-bifluorene]-9'-yl)phenyl)-9,9-dimethyl-N-phenyl-9H-fluorene-2-amine was introduced into another cell of said vacuum vapor depositing apparatus, and was evaporated by applying an electric current to the cell, thereby forming a second hole transport layer having a thickness of 60 nm on the first hole transport layer. Thereafter, host compounds as listed in Table 1 below was introduced into one cell of the vacuum vapor depositing apparatus as a host, and compound D-96 was introduced into another cell as a dopant. The two materials were deposited in a doping amount of 3 wt% (the amount of dopant) based on the total amount of the host and dopant to form a light-emitting layer having a thickness of 40 nm on the second hole transport layer. 2,4-bis(9,9-dimethyl-9H-fluoren-2-yl)-6-(naphthalen-2-yl)-1,3,5-triazine and lithium quinolate were then introduced into another two cells, evaporated at the same time, and deposited to form an electron transport layer having a thickness of 30 nm on the light-emitting layer. Next, after depositing lithium quinolate as an electron injection layer having a thickness of 2 nm on the electron transport layer, an AI cathode having a thickness of 80 nm was deposited by another vacuum vapor deposition apparatus on the electron injection layer. Thus, an OLED device was produced.

### Comparative Examples 1-1 to 1-3: Production of an organic electroluminescent device comprising a conventional organic electroluminescent material as a host

An OLED device was produced in the same manner as in the Device Examples above, except for using the compounds below as a host of the light-emitting material.

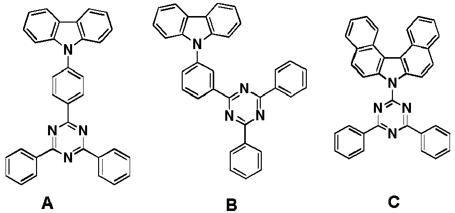

The evaluation results of the organic electroluminescent devices produced in Device Examples 1-1 to 1-12 and Comparative Examples 1-1 to 1-3 are shown in Table 1 as follows:

**[Table 1]**

| | **Host** | **Voltage (V)** | **Efficiency (cd/A)** | **Lifespan T98 (hr)** | **Color** |
|---|---|---|---|---|---|
| Device Example 1-1 | C-1 | 4.6 | 26.0 | 14 | Red |
| Device Example 1-2 | C-2 | 4.9 | 29.0 | 20 | Red |
| Device Example 1-3 | C-9 | 5.1 | 27.4 | 25 | Red |
| Device Example 1-4 | C-7 | 5.3 | 27.2 | 11 | Red |
| Device Example 1-5 | C-37 | 4.9 | 26.9 | 8 | Red |
| Device Example 1-6 | C-10 | 4.6 | 25.4 | 12 | Red |
| Device Example 1-7 | C-11 | 5.2 | 28.8 | 28 | Red |
| Device Example 1-8 | C-8 | 4.4 | 23.0 | 9 | Red |
| Device Example 1-9 | C-13 | 4.7 | 26.5 | 12 | Red |
| Device Example 1-10 | C-4 | 4.7 | 24.9 | 42 | Red |
| Device Example 1-11 | C-5 | 4.7 | 24.9 | 20 | Red |
| Device Example 1-12 | C-16 | 5.1 | 24.1 | 38 | Red |
| Comparative Example 1-1 | A | 4.6 | 23.2 | 0 | Red |
| Comparative Example 1-2 | B | 5.5 | 19.3 | 0 | Red |
| Comparative Example 1-3 | C | 5.4 | 24.0 | 2 | Red |

The data of driving voltage, efficiency, and color coordinate in Table 1 are based on 5,000 nit of luminance, and the lifespan data are time taken to be reduced from 100% to 98% of the luminance at 5,000 nit and a constant current.

### Comparative Example 2-1: Production of a blue light-emitting organic electroluminescent device not comprising an electron buffer layer

An OLED device was produced as follows. A transparent electrode indium tin oxide (ITO) thin film (10 Ω/sq) on a glass substrate for an organic light-emitting diode (OLED) device (Geomatec, Japan) was subjected to an ultrasonic washing with acetone, ethanol, and distilled water, sequentially, and was then stored in isopropanol. Next, the ITO substrate was mounted on a substrate holder of a vacuum vapor depositing apparatus. N⁴,N⁴'-diphenyl-N⁴,N^{4'}-bis(9-phenyl-9H-carbazol-3-yl)-[1,1'-biphenyl]-4,4'-diamine was introduced into a cell of said vacuum vapor depositing apparatus, and then the pressure in the chamber of said apparatus was controlled to 10⁻⁶ torr. Thereafter, an electric current was applied to the cell to evaporate the above introduced material, thereby forming a first hole injection layer having a thickness of 60 nm on the ITO substrate. 1,4,5,8,9,12-hexaazatriphenylene-hexacarbonitrile (HAT-CN) was then introduced into another cell of said vacuum vapor depositing apparatus, and was evaporated by applying an electric current to the cell, thereby forming a second hole injection layer having a thickness of 5 nm on the first hole injection layer. N-([1,1'-biphenyl]-4-yl)-9,9-dimethyl-N-(4-(9-phenyl-9H-carbazol-3-yl)phenyl)-9H-fluorene-2-amine was introduced into another cell of said vacuum vapor depositing apparatus, and was evaporated by applying an electric current to the cell, thereby forming a first hole transport layer having a thickness of 20 nm on the second hole injection layer. 9-(naphthalen-2-yl)-3-(4-(9-phenyl-9H-carbazol-3-yl)phenyl)-9H-carbazole as below (HT-2) was introduced into another cell of said vacuum vapor depositing apparatus, and was evaporated by applying an electric current to the cell, thereby forming a second hole transport layer having a thickness of 5 nm on the first hole transport layer. Thereafter, compound BH-1 as below was introduced into one cell of the vacuum vapor depositing apparatus as a host, and compound BD-1 as below was introduced into another cell as a dopant. The two materials were evaporated at different rates and were deposited in a doping amount of 2 wt% (the amount of dopant) based on the total amount of the host and dopant to form a light-emitting layer having a thickness of 20 nm on the second hole transport layer. 2-(3-(phenanthren-9-yl)-5-(pyridin-3-yl)phenyl)-4,6-diphenyl-1,3,5-triazine and lithium quinolate were then introduced into another two cells, evaporated at the same rate in an amount of 50 wt% each, and deposited to form an electron transport layer having a thickness of 35 nm on the light-emitting layer. Next, after depositing lithium quinolate as an electron injection layer having a thickness of 2 nm on the electron transport layer, an AI cathode having a thickness of 80 nm was deposited by another vacuum vapor deposition apparatus on the electron injection layer. Thus, an OLED device was produced. All the materials used for producing the OLED device were those purified by vacuum sublimation at 10⁻⁶ torr.

A driving voltage at 1,000 nit of luminance, CIE color coordinate, and time taken to be reduced from 100% to 90% of the luminance at 2,000 nit and a constant current of OLEDs are shown in Table 2 below.

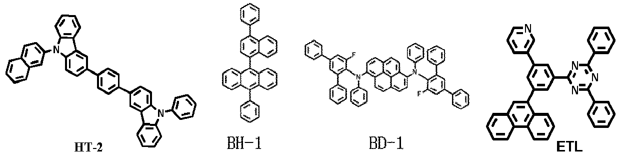

### Comparative Example 2-2, and Device Examples 2-1 and 2-2: Production of a blue light-emitting organic electroluminescent device according to the present invention

An OLED device was produced in the same manner as in Comparative Example 2-1, except for reducing the thickness of the electron transport layer to 30 nm, and inserting an electron buffer layer of 5 nm between the light-emitting layer and the electron transport layer, and evaluated. The evaluation results of the devices produced in Comparative Example 2-2, and Device Examples 2-1 and 2-2 are shown in Table 2 as follows:

**[Table 2]**

| | **Electron Buffer Layer** | **Voltage (V)** | **Color** | **Lifespan (%)** |
|---|---|---|---|---|
| Comparative Example 2-1 | - | 4.2 | Blue | 38.7 |
| Comparative Example 2-2 | C | 4.0 | Blue | 34.0 |
| Device Example 2-1 | C-1 | 4.6 | Blue | 57.8 |
| Device Example 2-2 | C-2 | 4.4 | Blue | 64.1 |

The blue fluorescent materials used at present have several problems. First, when exposed to high temperature during a process of producing a panel, a current characteristic of the blue fluorescent luminescent device changes to cause a problem of a change in luminance, and a drop of an interfacial characteristic between a light-emitting layer and an electron injection layer causes a decrease in luminance and lifespan. Second, in the case of devices comprising an anthracene based blue fluorescent host and a pyrene based dopant, an absolute value of a LUMO (lowest unoccupied molecular orbital) energy of the host (Ah) is higher than that of the dopant (Ad), and hole traps are magnified so that efficiency increases due to interfacial luminescence between the electron transport layer and the fluorescent light-emitting layer, but there is a problem in that the lifespan decreases.

Originally, LUMO energy and HOMO (highest occupied molecular orbital) energy levels have negative values. However, for convenience, the LUMO energy level and HOMO energy level are expressed in absolute values in the present invention. In addition, the values of the LUMO energy level are compared based on absolute values. In the present invention, Values measured by density functional theory (DFT) are used for the LUMO energy level and HOMO energy level.

In the blue organic electroluminescent device according to the present invention, by interposing an electron buffering layer, electron injection is controlled and interfacial characteristics between the light-emitting layer and the electron injection layer are improved, and thus lifespan characteristics are focused. Specifically, by interposing an electron buffer layer in the organic electroluminescent device, electron injection and transport can be controlled due to the difference of electron affinity according to LUMO energy level, between the light-emitting layer and the electron transport zone.

In the organic electroluminescent device according to the present invention, the LUMO energy level of the electron buffer layer may be higher than the LUMO energy level of the host compound. Specifically, the difference in the LUMO energy levels between the electron buffer layer and the host compound may be 0.4 eV or less. For example, the LUMO energy levels of the electron buffer layer and the host compound may be 2.0 eV and 1.6 eV, respectively, and thus the difference in the LUMO energy levels may be 0.4 eV. Due to such LUMO barrier between the host compound and the electron buffer layer, improved lifespan characteristics can be shown compared to a device not comprising an electron buffer layer. This is because a drop of an interfacial characteristic within the device is mitigated through electron injection control effect due to the electron buffer layer.

As shown in Table 2, due to the electron injection control effect of the electron buffer layer of the present invention, Device Examples 2-1 and 2-2 showed better lifespan characteristics than Comparative Examples 2-1 and 2-2 which do not comprise an electron buffer layer. These results can be effectively used henceforth in the field of flexible display or lighting device which require long lifespan.

## Claims

1. An organic electroluminescent device comprising an organic electroluminescent compound represented by the following formula 1 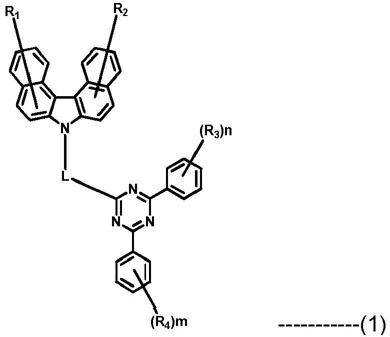 wherein
L represents a substituted or unsubstituted (C6-C30)arylene;
R₁ to R₂ each independently represent hydrogen, deuterium, a halogen, a cyano, a substituted or unsubstituted (C1-C30)alkyl, a substituted or unsubstituted (C6-C30)aryl, a substituted or unsubstituted 3- to 30-membered heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di- (C1-C30)alkylamino, a substituted or unsubstituted mono- or di- (C6-C30)arylamino, or a substituted or unsubstituted (C1-C30)alkyl(C6-C30)arylamino;
R₃ to R₄ each independently represent hydrogen, deuterium, a halogen, a cyano, a substituted or unsubstituted (C1-C30)alkyl, a substituted or unsubstituted (C6-C30)aryl, a substituted or unsubstituted 3- to 30-membered heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di- (C1-C30)alkylamino, a substituted or unsubstituted mono- or di- (C6-C30)arylamino, or a substituted or unsubstituted (C1-C30)alkyl(C6-C30)arylamino; or are linked to an adjacent substituent(s) to form a substituted or unsubstituted, mono- or polycyclic, (C3-C30) alicyclic or aromatic ring, whose carbon atom(s) may be replaced with at least one hetero atom selected from nitrogen, oxygen, and sulfur;
the heteroaryl contains at least one hetero atom selected from B, N, O, S, Si, and P; and
n and m represent an integer of 0 to 3.

2. The organic electroluminescent device according to claim 1, wherein the substituents of the substituted (C1-C30)alkyl, the substituted (C6-C30)aryl, the substituted 3- to 30-membered heteroaryl, the substituted (C3-C30)cycloalkyl, the substituted (C1-C30)alkoxy, the substituted tri(C1-C30)alkylsilyl, the substituted di(C1-C30)alkyl(C6-C30)arylsilyl, the substituted (C1-C30)alkyldi(C6-C30)arylsilyl, the substituted tri(C6-C30)arylsilyl, the substituted mono- or di- (C1-C30)alkylamino, the substituted mono- or di-(C6-C30)arylamino, the substituted (C1-C30)alkyl(C6-C30)arylamino, and the substituted mono- or polycyclic, (C3-C30) alicyclic or aromatic ring in R₁ to R₄ each independently are at least one selected from the group consisting of deuterium, a halogen, a cyano, a carboxyl, a nitro, a hydroxyl, a (C1-C30)alkyl, a halo(C1-C30)alkyl, a (C2-C30) alkenyl, a (C2-C30) alkynyl, a (C1-C30)alkoxy, a (C1-C30)alkylthio, a (C3-C30)cycloalkyl, a (C3-C30)cycloalkenyl, a 3- to 7-membered heterocycloalkyl, a (C6-C30)aryloxy, a (C6-C30)arylthio, a 5- to 30-membered heteroaryl unsubstituted or substituted with a (C6-C30)aryl, a (C6-C30)aryl unsubstituted or substituted with a 5- to 30-membered heteroaryl, a tri(C1-C30)alkylsilyl, a tri(C6-C30)arylsilyl, a di(C1-C30)alkyl(C6-C30)arylsilyl, a (C1-C30)alkyldi(C6-C30)arylsilyl, an amino, a mono- or di- (C1-C30)alkylamino, a mono- or di-(C6-C30)arylamino, a (C1-C30)alkyl(C6-C30)arylamino, a (C1-C30)alkylcarbonyl, a (C1-C30)alkoxycarbonyl, a (C6-C30)arylcarbonyl, a di(C6-C30)arylboronyl, a di(C1-C30)alkylboronyl, a (C1-C30)alkyl(C6-C30)arylboronyl, a (C6-C30)aryl(C1-C30)alkyl, and a (C1-C30)alkyl(C6-C30)aryl.

3. The organic electroluminescent device according to claim 1, wherein L represents a substituted or unsubstituted (C6-C20)arylene;
R₁ to R₂ each independently represent hydrogen, or a substituted or unsubstituted (C6-C20)aryl;
and R₃ to R₄ each independently represent hydrogen, or a substituted or unsubstituted (C6-C20)aryl, or are linked to an adjacent substituent(s) to form a substituted or unsubstituted, mono- or polycyclic, (C6-C20) alicyclic or aromatic ring, whose carbon atom(s) may be replaced with at least one hetero atom selected from nitrogen, oxygen, and sulfur.

4. The organic electroluminescent device according to claim 1, wherein L represents
a (C6-C12)arylene unsubstituted or substituted with a (C1-C6)alkyl or a (C6-C12)aryl; R₁ to R₂ each independently represent hydrogen or an unsubstituted (C6-C12)aryl;
and R₃ to R₄ each independently represent hydrogen or an unsubstituted (C6-C12)aryl, or are linked to an adjacent substituent(s) to form a mono- or polycyclic, (C6-C15) alicyclic or aromatic ring unsubstituted or substituted with a (C1-C6)alkyl or a (C6-C12)aryl, whose carbon atom(s) may be replaced with at least one hetero atom selected from nitrogen, oxygen, and sulfur.

5. The organic electroluminescent device according to claim 1, wherein the compound represented by formula 1 is selected from the group consisting of:

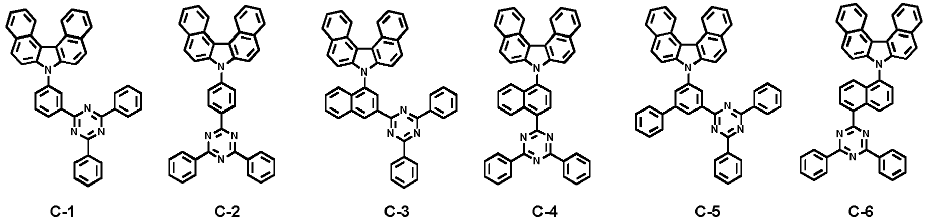

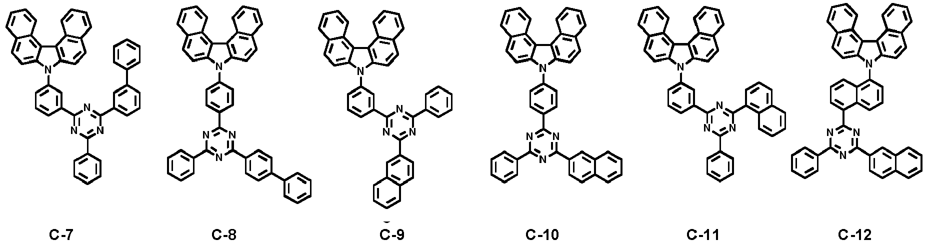

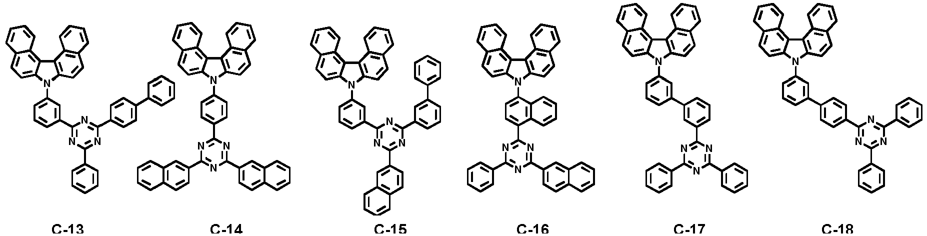

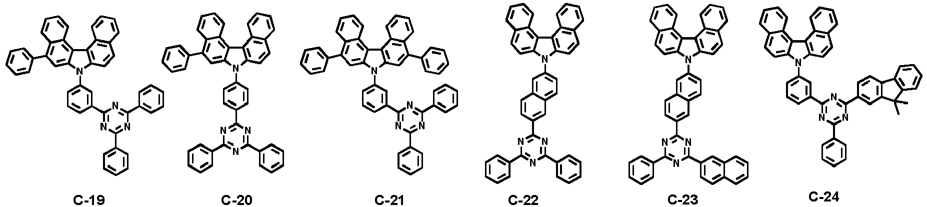

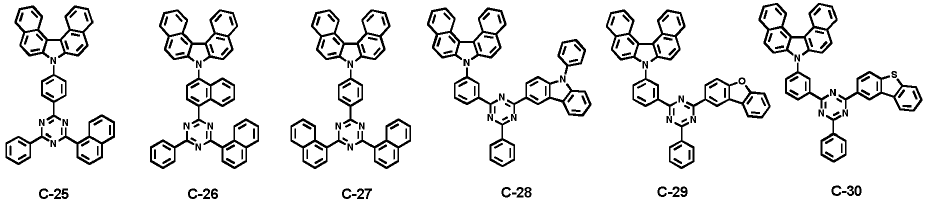

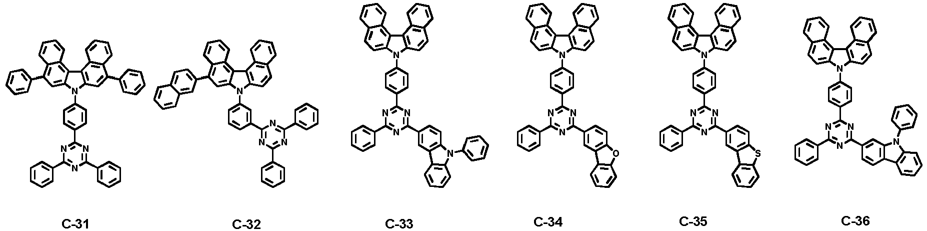

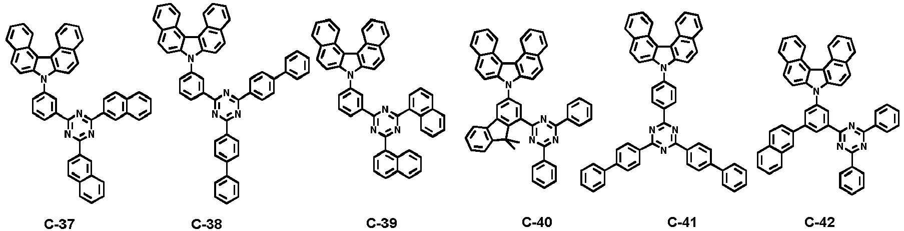

## Patentansprüche

1. Organische Elektrolumineszenzvorrichtung, umfassend eine organische elektrolumineszente Verbindung, dargestellt durch die folgende Formel 1 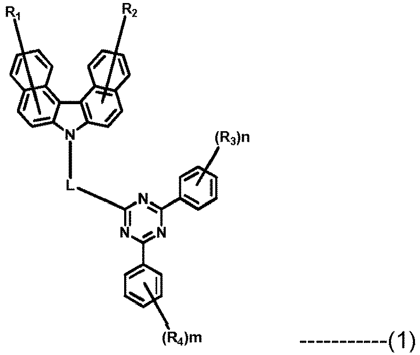 wobei
L ein substituiertes oder unsubstituiertes (C6-C30)Arylen darstellt;
R₁ bis R₂ jeweils unabhängig Wasserstoff, Deuterium, ein Halogen, ein Cyano, ein substituiertes oder unsubstituiertes (C1-C30)Alkyl, ein substituiertes oder unsubstituiertes (C6-C30)Aryl, ein substituiertes oder unsubstituiertes 3- bis 30-gliedriges Heteroaryl, ein substituiertes oder unsubstituiertes (C3-C30)Cycloalkyl, ein substituiertes oder unsubstituiertes (C1-C30)Alkoxy, ein substituiertes oder unsubstituiertes Tri(Cl-C30)alkylsilyl, ein substituiertes oder unsubstituiertes Di(C1-C30)alkyl(C6-C30)arylsilyl, ein substituiertes oder unsubstituiertes (Cl-C30)Alkyldi(C6-C30)arylsilyl, ein substituiertes oder unsubstituiertes Tri(C6-C30)arylsilyl, ein substituiertes oder unsubstituiertes Mono- oder Di(C1-C30)alkylamino, ein substituiertes oder unsubstituiertes Mono- oder Di(C6-C30)arylamino, oder ein substituiertes oder unsubstituiertes (Cl-C30)Alkyl(C6-C30)arylamino darstellen;
R₃ bis R₄ jeweils unabhängig Wasserstoff, Deuterium, ein Halogen, ein Cyano, ein substituiertes oder unsubstituiertes (Cl-C30)Alkyl, ein substituiertes oder unsubstituiertes (C6-C30)Aryl, ein substituiertes oder unsubstituiertes 3- bis 30-gliedriges Heteroaryl, ein substituiertes oder unsubstituiertes (C3-C30)Cycloalkyl, ein substituiertes oder unsubstituiertes (C1-C30)Alkoxy, ein substituiertes oder unsubstituiertes Tri(Cl-C30)alkylsilyl, ein substituiertes oder unsubstituiertes Di(C1-C30)alkyl(C6-C30)arylsilyl, ein substituiertes oder unsubstituiertes (Cl-C30)Alkyldi(C6-C30)arylsilyl, ein substituiertes oder unsubstituiertes Tri(C6-C30)arylsilyl, ein substituiertes oder unsubstituiertes Mono- oder Di(C1-C30)alkylamino, ein substituiertes oder unsubstituiertes Mono- oder Di(C6-C30)arylamino, oder ein substituiertes oder unsubstituiertes (Cl-C30)Alkyl(C6-C30)arylamino darstellen; oder mit einem oder mehreren benachbarten Substituenten zum Bilden eines substituierten oder unsubstituierten, mono- oder polyzyklischen, (C3-C30)alizyklischen oder aromatischen Rings verbunden sind, dessen Kohlenstoffatom(e) durch mindestens ein Heteroatom ersetzt werden kann/können, ausgewählt aus Stickstoff, Sauerstoff und Schwefel;
das Heteroaryl mindestens ein Heteroatom enthält, ausgewählt aus B, N, O, S, Si und P; und
n und m eine Ganzzahl von 0 bis 3 darstellen.

2. Organische Elektrolumineszenzvorrichtung nach Anspruch 1, wobei die Substituenten des substituierten (C1-C30)Alkyls, des substituierten (C6-C30)Aryls, des substituierten 3- bis 30-gliedrigen Heteroaryls, des substituierten (C3-C30)Cycloalkyls, des substituierten (C1-C30)Alkoxys, des substituierten Tri(C1-C30)alkylsilyls, der substituierte Di(C1-C30)alkyl(C6-C30)arylsilyls, des substituierten (C1-C30)Alkyldi(C6-C30)arylsilyls, des substituierten Tri(C6-C30)arylsilyls, des substituierten Mono- oder Di(C1-C30)alkylaminos, des substituierten Mono- oder Di(C6-C30)arylaminos, des substituierten (Cl-C30)Alkyl(C6-C30)arylaminos und des substituierten mono- oder polyzyklischen, (C3-C30)alizyklischen oder aromatischen Rings in R₁ bis R₄ jeweils unabhängig mindestens eines sind, gewählt aus der Gruppe, bestehend aus Deuterium, einem Halogen, einem Cyano, einem Carboxyl, einem Nitro, einem Hydroxyl, einem (C1-C30)Alkyl, einem Halo(C1-C30)alkyl, einem (C2-C30)Alkenyl, einem (C2-C30)Alkynyl, einem (C1-C30)Alkoxy, einem (C1-C30)Alkylthio, einem (C3-C30)Cycloalkyl, einem (C3-C30)Cycloalkenyl, einem 3- bis 7-gliedrigen Heterocycloalkyl, einem (C6-C30)Aryloxy, einem (C6-C30)Arylthio, einem 5- bis 30-gliedrigen Heteroaryl, welches unsubstituiert oder mit einem (C6-C30)Aryl substituiert ist, einem (C6-C30)Aryl, welches unsubstituiert oder mit einem 5- bis 30-gliedrigen Heteroaryl substituiert ist, einem Tri(C1-C30)alkylsilyl, einem Tri(C6-C30)arylsilyl, einem Di(C1-C30)alkyl(C6-C30)arylsilyl, einem (Cl-C30)Alkyldi(C6-C30)arylsilyl, einem Amino, einem Mono- oder Di(C1-C30)alkylamino, einem Mono- oder Di(C6-C30)arylamino, einem (C1-C30)Alkyl(C6-C30)arylamino, einem (C1-C30)Alkylcarbonyl, einem (C1-C30)Alkoxycarbonyl, einem (C6-C30)Arylcarbonyl, einem Di(C6-C30)arylboronyl, einem Di(C1-C30)alkylboronyl, einem (Cl-C30)Alkyl(C6-C30)arylboronyl, einem (C6-C30)Aryl(Cl-C30)alkyl und einem (C1-C30)Alkyl(C6-C30)aryl.

3. Organische Elektrolumineszenzvorrichtung nach Anspruch 1, wobei L ein substituiertes oder unsubstituiertes (C6-C20)Arylen darstellt;
R₁ bis R₂ jeweils unabhängig Wasserstoff oder ein substituiertes oder unsubstituiertes (C6⁻C20)Aryl darstellen;
und R₃ bis R₄ jeweils unabhängig Wasserstoff oder ein substituiertes oder unsubstituiertes (C6-C20)Aryl darstellen, oder mit einem oder mehreren benachbarten Substituenten verbunden sind, um einen substituierten oder unsubstituierten, mono- oder polyzyklischen, (C6-C20)alizyklischen oder aromatischen Ring zu bilden, dessen Kohlenstoffatom(e) durch mindestens ein Heteroatom ersetzt werden kann/können, ausgewählt aus Stickstoff, Sauerstoff und Schwefel.

4. Organische Elektrolumineszenzvorrichtung nach Anspruch 1, wobei L ein (C6-C12)Arylen darstellt, welches unsubstituiert oder mit einem (C1-C6)Alkyl oder einem (C6-C12)Aryl substituiert ist;
R₁ bis R₂ jeweils unabhängig Wasserstoff oder ein unsubstituiertes (C6-C12)Aryl darstellen;
und R₃ bis R₄ jeweils unabhängig Wasserstoff oder ein unsubstituiertes (C6-C12)Aryl darstellen, oder mit einem oder mehreren benachbarten Substituenten verbunden sind, um einen mono- oder polyzyklischen, (C6-C15)alizyklischen oder aromatischen Ring zu bilden, welcher unsubstituiert oder mit einem (C1-C6)Alkyl oder einem (C6-C12)Aryl substituiert ist, dessen Kohlenstoffatom(e) durch mindestens ein Heteroatom ersetzt werden kann/können, ausgewählt aus Stickstoff, Sauerstoff und Schwefel.

5. Organische Elektrolumineszenzvorrichtung nach Anspruch 1, wobei die durch die Formel 1 dargestellte Verbindung aus der Gruppe ausgewählt ist, bestehend aus:

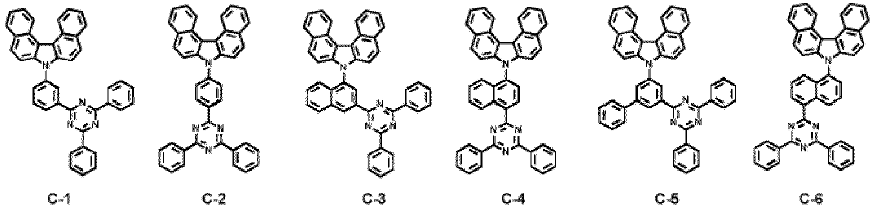

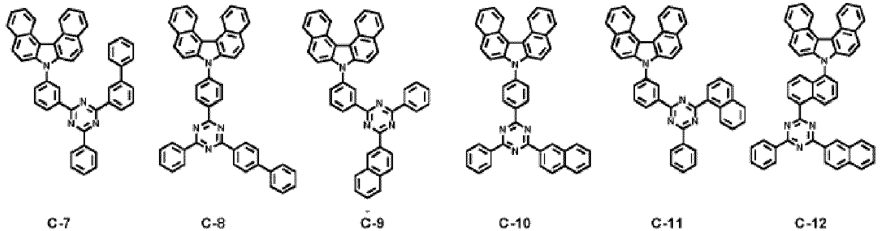

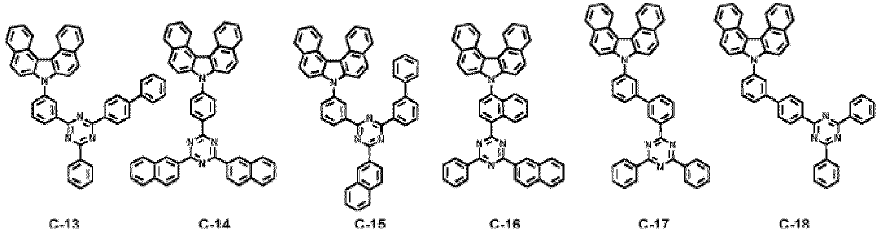

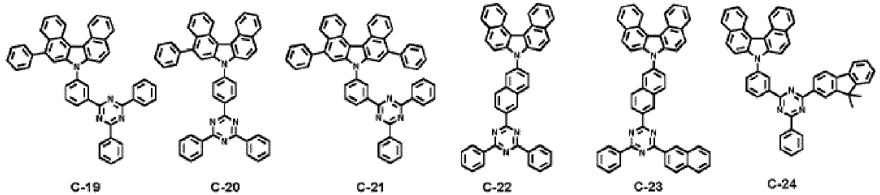

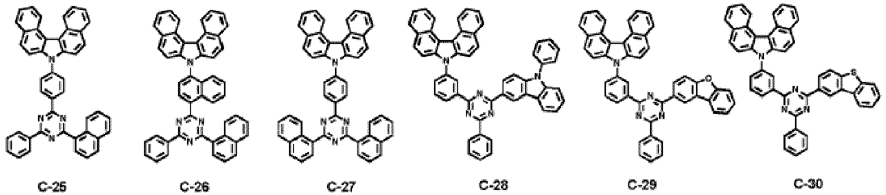

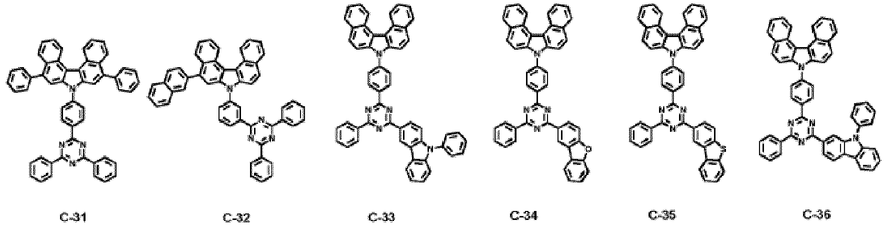

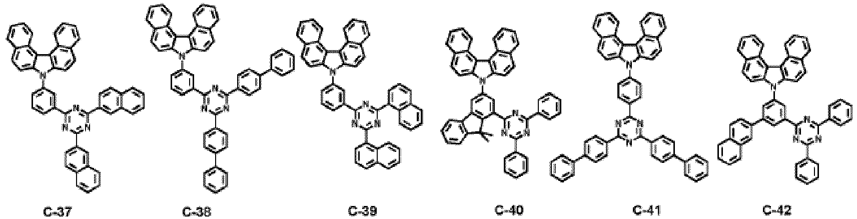

## Revendications

1. Dispositif électroluminescent organique comprenant un composé électroluminescent organique représenté par la formule 1 suivante : 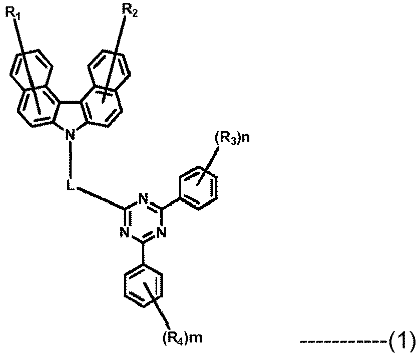 dans laquelle
L représente un arylène en (C6-C30) substitué ou non substitué ;
R₁ à R₂ représentent chacun indépendamment un hydrogène, un deutérium, un halogène, un cyano, un alkyle en (C1-C30) substitué ou non substitué, un aryle en (C6-C30) substitué ou non substitué, un hétéroaryle à 3 à 30 éléments substitué ou non substitué, un cycloalkyle en (C3-C30) substitué ou non substitué, un alcoxy en (C1-C30) substitué ou non substitué, un tri-alkylsilyle en (C1-C30) substitué ou non substitué, un di-alkyle en (Cl-C30)-arylsilyle en (C6-C30) substitué ou non substitué, un alkyle en (C1-C30)-diarylsilyle en (C6-C30) substitué ou non substitué, un tri-arylsilyle en (C6-C30) substitué ou non substitué, un mono-ou di-alkylamino en (C1-C30) substitué ou non substitué, un mono- ou di-arylamino en (C6-C30) substitué ou non substitué, ou un alkyle en (C1-C30)-arylamino en (C6-C30) substitué ou non substitué ;
R3 à R4 représentent chacun indépendamment un hydrogène, un deutérium, un halogène, un cyano, un alkyle en (C1-C30) substitué ou non substitué, un aryle en (C6-C30) substitué ou non substitué, un hétéroaryle à 3 à 30 éléments substitué ou non substitué, un cycloalkyle en (C3-C30) substitué ou non substitué, un alcoxy en (C1-C30) substitué ou non substitué, un tri-alkylsilyle en (C1-C30) substitué ou non substitué, un di-alkyle en (Cl-C30)-arylsilyle en (C6-C30) substitué ou non substitué, un alkyle en (C1-C30)-diarylsilyle en (C6-C30) substitué ou non substitué, un tri-arylsilyle en (C6-C30) substitué ou non substitué, un mono-ou di-alkylamino en (C1-C30) substitué ou non substitué, un mono- ou di-arylamino en (C6-C30) substitué ou non substitué, ou un alkyle en (C1-C30)-arylamino en (C6-C30) substitué ou non substitué ; ou sont liés à un ou plusieurs substituant(s) adjacent(s) pour former un cycle alicyclique ou aromatique en (C3-C30), mono- ou polycyclique, substitué ou non substitué, dont l'atome ou les atomes de carbone peut/peuvent être remplacé(s) par au moins un hétéroatome sélectionné parmi l'azote, l'oxygène et le soufre ;
l'hétéroaryle contient au moins un hétéroatome sélectionné parmi B, N, O, S, Si et P ; et
n et m représentent un nombre entier de 0 à 3.

2. Dispositif électroluminescent organique selon la revendication 1, dans lequel les substituants de l'alkyle en (C1-C30) substitué, de l'aryle en (C6-C30) substitué, de l'hétéroaryle à 3 à 30 éléments substitué, du cycloalkyle en (C3-C30) substitué, de l'alcoxy en (C1-C30) substitué, du tri-alkylsilyle en (C1-C30) substitué, du di-alkyle en (C1-C30)-arylsilyle en (C6-C30) substitué, de l'alkyle en (Cl-C30)-diarylsilyle en (C6-C30) substitué, du tri-arylsilyle en (C6-C30) substitué, du mono-ou di-alkylamino en (C1-C30) substitué, du mono- ou di-arylamino en (C6-C30) substitué, de l'alkyle en (C1-C30)-arylamino en (C6-C30) substitué, et du cycle alicyclique ou aromatique en (C3-C30), mono- ou polycyclique, substitué dans R₁ à R₄ représentent chacun indépendamment au moins un élément sélectionné dans le groupe constitué de deutérium, d'un halogène, d'un cyano, d'un carboxyle, d'un nitro, d'un hydroxyle, d'un alkyle en (C1-C30), d'un halo-alkyle en (C1-C30), d'un alcényle en (C2-C30), d'un alcynyle en (C2-C30), d'un alcoxy en (C1-C30), d'un alkylthio en (C1-C30), d'un cycloalkyle en (C3-C30), d'un cycloalcényle en (C3-C30), d'un hétérocycloalkyle à 3 à 7 éléments, d'un aryloxy en (C6-C30), d'un arylthio en (C6-C30), d'un hétéroaryle à 5 à 30 éléments non substitué ou substitué avec un aryle en (C6-C30), un aryle en (C6-C30), non substitué ou substitué par un hétéroaryle à 5 à 30 éléments, un tri-alkylsilyle en (C1-C30), un tri-arylsilyle en (C6-C30), un di-alkyle en (Cl-C30)-arylsilyle en (C6-C30), un alkyle en (Cl-C30)-diarylsilyle en (C6-C30), un amino, un mono- ou di-alkylamino en (C1-C30), un mono- ou di-arylamino en (C6-C30), un alkyle en (Cl-C30)-arylamino en (C6-C30), un alkylcarbonyle en (C1-C30), un alcoxycarbonyle en (C1-C30), un arylcarbonyle en (C6-C30), un di-arylboronyle en (C6-C30), un di-alkylboronyle en (C1-C30), un alkyle en (C1-C30)-arylboronyle en (C6-C30), un aryle en (C6-C30)-alkyle en (C1-C30), et un alkyle en (C1-C30)-aryle en (C6-C30).

3. Dispositif électroluminescent organique selon la revendication 1, dans lequel L représente un arylène en (C6-C20) substitué ou non substitué ;
R₁ à R₂ représentent chacun indépendamment un hydrogène, ou un aryle en (C6-C20) substitué ou non substitué ;
et R₃ à R₄ représentent chacun indépendamment un hydrogène, ou un aryle en (C6-C20) substitué ou non substitué, ou sont liés à un ou plusieurs substituants adjacents pour former un cycle alicyclique ou aromatique en (C6-C20), mono- ou polycyclique, substitué ou non substitué, dont l'atome ou les atomes de carbone peut/peuvent être remplacé(s) par au moins un hétéroatome sélectionné parmi l'azote, l'oxygène et le soufre.

4. Dispositif électroluminescent organique selon la revendication 1, dans lequel L représente un arylène en (C6-C20) non substitué substitué avec un alkyle en (C1-C6) ou un aryle en (C6-C12) ;
R₁ à R₂ représentent chacun indépendamment un hydrogène, ou un aryle en (C6-C12) non substitué,
et R₃ à R₄ représentent chacun indépendamment un hydrogène ou un aryle en (C6-C12) non substitué, ou sont liés à un ou plusieurs substituants adjacents pour former un cycle alicyclique ou aromatique en (C6-C15), mono- ou polycyclique, non substitué ou substitué avec un alkyle en (C1-C6) ou un aryle en (C6-C12), dont l'atome ou les atomes de carbone peut/peuvent être remplacé(s) par au moins un hétéroatome sélectionné parmi l'azote, l'oxygène et le soufre.

5. Dispositif électroluminescent organique selon la revendication 1, dans lequel le composé représenté par la formule 1 est sélectionné dans le groupe constitué par :
